(19) Europäisches Patentamt
European Patent Office
Office européen des brevets

(11) EP 1 873 248 A1

(12) EUROPEAN PATENT APPLICATION

(43) Date of publication:
**02.01.2008 Bulletin 2008/01**

(21) Application number: **06013092.9**

(22) Date of filing: **26.06.2006**

(51) Int Cl.:
***C12N 15/61*** *(2006.01)* ***C12N 9/92*** *(2006.01)*
***C12P 17/04*** *(2006.01)* ***C12P 1/04*** *(2006.01)*
***C12N 1/20*** *(2006.01)*

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **DSM IP Assets B.V.**
**6411 TE Heerlen (NL)**

(72) Inventor: **The designation of the inventor has not yet been filed**

(74) Representative: **Pressner, Dietmar et al**
**DSM Nutritional Products Ltd**
**Wurmisweg 576**
**4303 Kaiseraugst (CH)**

(54) **Biological process using a glucose-6-phosphate isomerase**

(57) The present invention relates to microorganisms genetically engineered to reduce carbon source diversion throughout the bioconversion of a carbon source, such as e.g. sorbitol, into biomass. Processes for generating such microorganisms are also provided by the present invention. The invention also relates to polynucleotide sequences comprising genes that encode proteins that are involved in the bioconversion of a carbon source such as e.g. sorbitol, into biomass. The invention also features polynucleotides comprising the full-length polynucleotide sequences of the novel genes and fragments thereof, the novel polypeptides encoded by the polynucleotides and fragments thereof, as well as their functional equivalents. Also included are processes of using the polynucleotides and modified polynucleotide sequences to transform host microorganisms leading to a microorganism with reduced carbon source diversion, i.e. higher yield and/or efficiency of biomass production from a carbon source such as e.g. sorbitol.

EP 1 873 248 A1

SEQUENCE LISTING

<110> DSM IP Assets B.V.

<120> Novel biological process

<130> 25662

<160> 15

<170> PatentIn version 3.2

<210> 1
<211> 1704
<212> DNA
<213> Gluconobacter oxydans DSM 2343

<400> 1
```
ctgcgggtca gcgccgacct tccgccccgat ctgcgtgatg acgttgcagc ggccgaaaag      60

gactggagcc atagcggtgg gctgcgccgc atctggaaga aggacccgtc cgtctggacg     120

aatggtccgg aagccgggtg gctgaactgg ctgacggtcg tgcaggatcg tctgtggcat     180

atagaggagc tggaagttct ggctcgcgat gtccgcagtc gcggcttcaa ggacatcctg     240

cttctgggca tgggcggttc gagccttggt cccgaggtgc tcgccgagac gttcggaaag     300

cgcgaaggct ggccgaagct gcatgtgctc gacagcacgg acccgcagca ggttacggcg     360

ttcgaaaagg ccatcgatcc gaagaacacg ctgttcatcg tggccagcaa gtccggcggc     420

acgctggagc cgaacatcct gttcgcgcat ttctggcagg ttgcagggca ggctctgggc     480

aagaagccgg gtgacagctt catcgccatc accgatccgg gctcgcacat gcagaaggtg     540

gccgaggaaa acggcttctg gcgcattttc tacggcgatc cgaaaattgg tggccgctat     600

tcggttctgt ccaacttcgg tctggtcccg gcagctgcga gcggcattga tgtccgcaag     660

ctgctgacgg tcacgcgcct gatggtggaa tcctgtgacg gaagcgcgcc gccttccgcc     720

aatccgggtg cggttctggg cctgatcctg ggacatgccg cgcactgctt taatcgtgac     780

aaggtcacga tcctggcgtc cgaaggcatt gcctcgcttg ggcctggct ggagcagttg      840

atcgccgaga gcacgggtaa gaaaggtctg ggcctcatcc ccatcgatga ggaagagctt     900

ggaacgccgg atgtctacgg aacggaccgt gtcttcgtgg atctgctgct gggcgacgat     960

gttcccgaca gccgtcttgg ggccctgcgt gctgctggtg caccggtggt caccatccgt    1020

ctggcggaca aggcccagat cggacaggcg ttcttcctgt tcgagttcgc caccgccgtg    1080

gcgggtgcgg ttctggggat cgatccgttc gatcagccgg atgtggagtt cagcaagctt    1140

gagacgcgca aactgacttc ggcctatgcc gagacgggat cgctgccgcc ggaaacgccc    1200

ttcgcaaagg atggtccgct gtccttctat gcggatgcga agaacacgga agcctctgga    1260
```

aacaaaggcg acgcggtctc catcctcaag gcgctctttg atcgcgtaaa gccgggtgac 1320

tatgtcgggc tgctcgccta tatcaggcgc gaccagcaga cacgggactg ggcacagtcc 1380

gttcgcatgg agcttcggga tgctctgaag gtggcaacag ctgccgaatt cggaccccgg 1440

ttcctgcatt cgacgggcca ggcctacaag ggcggacccg acagtggcgt gttcctgcag 1500

gttacggcga cggatgcggc ggatgtgccg gttccgggcc atggcttcag cttcagtgtc 1560

gtgaaagcgg cacaggcgcg aggcgatttc gaggtgctcg cggcccgtgg tcgtcgtgcc 1620

ctccgcgttc atatcgatgg accgctggag gacgggctga aggctctgga aacggcgatc 1680

cacaaagcac tggcaggagc ataa 1704

<210> 2
<211> 567
<212> PRT
<213> Gluconobacter oxydans DSM 2343

<400> 2

Leu Arg Val Ser Ala Asp Leu Pro Pro Asp Leu Arg Asp Asp Val Ala
1 5 10 15

Ala Ala Glu Lys Asp Trp Ser His Ser Gly Gly Leu Arg Arg Ile Trp
20 25 30

Lys Lys Asp Pro Ser Val Trp Thr Asn Gly Pro Glu Ala Gly Trp Leu
35 40 45

Asn Trp Leu Thr Val Val Gln Asp Arg Leu Trp His Ile Glu Glu Leu
50 55 60

Glu Val Leu Ala Arg Asp Val Arg Ser Arg Gly Phe Lys Asp Ile Leu
65 70 75 80

Leu Leu Gly Met Gly Gly Ser Ser Leu Gly Pro Glu Val Leu Ala Glu
85 90 95

Thr Phe Gly Lys Arg Glu Gly Trp Pro Lys Leu His Val Leu Asp Ser
100 105 110

Thr Asp Pro Gln Gln Val Thr Ala Phe Glu Lys Ala Ile Asp Pro Lys
115 120 125

Asn Thr Leu Phe Ile Val Ala Ser Lys Ser Gly Gly Thr Leu Glu Pro
130 135 140

```
Asn Ile Leu Phe Ala His Phe Trp Gln Val Ala Gly Gln Ala Leu Gly
145                 150                 155                 160

Lys Lys Pro Gly Asp Ser Phe Ile Ala Ile Thr Asp Pro Gly Ser His
                165                 170                 175

Met Gln Lys Val Ala Glu Glu Asn Gly Phe Trp Arg Ile Phe Tyr Gly
            180                 185                 190

Asp Pro Lys Ile Gly Gly Arg Tyr Ser Val Leu Ser Asn Phe Gly Leu
        195                 200                 205

Val Pro Ala Ala Ala Ser Gly Ile Asp Val Arg Lys Leu Leu Thr Val
    210                 215                 220

Thr Arg Leu Met Val Glu Ser Cys Asp Gly Ser Ala Pro Pro Ser Ala
225                 230                 235                 240

Asn Pro Gly Ala Val Leu Gly Leu Ile Leu Gly His Ala Ala His Cys
                245                 250                 255

Phe Asn Arg Asp Lys Val Thr Ile Leu Ala Ser Glu Gly Ile Ala Ser
            260                 265                 270

Leu Gly Ala Trp Leu Glu Gln Leu Ile Ala Glu Ser Thr Gly Lys Lys
        275                 280                 285

Gly Leu Gly Leu Ile Pro Ile Asp Glu Glu Glu Leu Gly Thr Pro Asp
    290                 295                 300

Val Tyr Gly Thr Asp Arg Val Phe Val Asp Leu Leu Leu Gly Asp Asp
305                 310                 315                 320

Val Pro Asp Ser Arg Leu Gly Ala Leu Arg Ala Ala Gly Ala Pro Val
                325                 330                 335

Val Thr Ile Arg Leu Ala Asp Lys Ala Gln Ile Gly Gln Ala Phe Phe
            340                 345                 350

Leu Phe Glu Phe Ala Thr Ala Val Ala Gly Ala Val Leu Gly Ile Asp
        355                 360                 365

Pro Phe Asp Gln Pro Asp Val Glu Phe Ser Lys Leu Glu Thr Arg Lys
```

370 375 380

Leu Thr Ser Ala Tyr Ala Glu Thr Gly Ser Leu Pro Pro Glu Thr Pro
385 390 395 400

Phe Ala Lys Asp Gly Pro Leu Ser Phe Tyr Ala Asp Ala Lys Asn Thr
405 410 415

Glu Ala Ser Gly Asn Lys Gly Asp Ala Val Ser Ile Leu Lys Ala Leu
420 425 430

Phe Asp Arg Val Lys Pro Gly Asp Tyr Val Gly Leu Leu Ala Tyr Ile
435 440 445

Arg Arg Asp Gln Gln Thr Arg Asp Trp Ala Gln Ser Val Arg Met Glu
450 455 460

Leu Arg Asp Ala Leu Lys Val Ala Thr Ala Ala Glu Phe Gly Pro Arg
465 470 475 480

Phe Leu His Ser Thr Gly Gln Ala Tyr Lys Gly Gly Pro Asp Ser Gly
485 490 495

Val Phe Leu Gln Val Thr Ala Thr Asp Ala Ala Asp Val Pro Val Pro
500 505 510

Gly His Gly Phe Ser Phe Ser Val Val Lys Ala Ala Gln Ala Arg Gly
515 520 525

Asp Phe Glu Val Leu Ala Ala Arg Gly Arg Arg Ala Leu Arg Val His
530 535 540

Ile Asp Gly Pro Leu Glu Asp Gly Leu Lys Ala Leu Glu Thr Ala Ile
545 550 555 560

His Lys Ala Leu Ala Gly Ala
565

<210> 3
<211> 31
<212> DNA
<213> Artificial

<220>
<223> PCR primer

<400> 3
taagcattta aatctgcggg tcagcgccga c 31

<210> 4
<211> 35
<212> DNA
<213> Artificial

<220>
<223> PCR primer

<400> 4
taagcattta aatttatgct cctgccagtg ctttg 35

<210> 5
<211> 1704
<212> DNA
<213> Gluconobacter oxydans DSM 17078

<400> 5
ctgcgggtta gcgcggatct tccgccggat ctgcgtgacg atgtggccgg agcggaaaag 60

gactggagcc atagtggcgg tctgcgtcgc atctggaaga aggacccgtc cgtctggacg 120

aatggtccgg aagccgggtg gctgaactgg ctgacggttg tgcaggatcg tctgtggcat 180

attgaggagc tggaagttct ggcccgcgat gtccgcagtc gcggcttcaa ggatatcctg 240

cttctgggta tgggcggttc gagccttggt cccgaggtgc tcgctgagac tttcggaaag 300

cgcgaaggct ggccgaagct gcatgtgctc gacagcacgg acccgcagca ggttacggcg 360

ttcgaaaagg ctattgatcc gaagaacacg ctgttcatcg tggccagcaa gtccggcggt 420

acgctggagc cgaacatctt gttcgcgcat ttctggcagg ttgcgggaca ggctctgggc 480

aagaagccgg gtgacagctt catcgccatc accgatccgg gctcgcacat gcagaaggtg 540

gccgaggaaa acggcttctg gcgcattttc tacggcgatc cgaaaatcgg cggccgttat 600

tcggtcctgt ccaacttcgg tctggtcccg gcagctgcga gcggcattga tgtccgcaag 660

ctgctgacgg tcacgcgcct gatggtggaa tcctgtgacg gaagcgcacc gccttccgcc 720

aatccgggtg cggtccttgg tctgatcctg ggacatgccg cgcactgctt taatcgtgac 780

aaggtcacga tcctggcgtc cgaaggcatt gcctcgctgg gtgcctggct ggaacagctg 840

attgcagaga gcaccggcaa gaagggtctc ggcctcatcc ccatcgatga ggaagagctt 900

ggaacgccgg atgtctacgg aacggaccgc gtcttcgtgg atctgctgct gggcgacgat 960

gttcctgaca gccgccttgg tgccctgcgt gctgctggtg caccagtggt caccatccgt 1020

ctggcggaca aggcccagat cgggcaggcg ttcttcctat tcgagttcgc cacggccgtg 1080

gcgggtgcgg ttctggggat cgatccgttc gatcagccgg atgtggagtt cagcaagctc 1140

gagacgcgca agctgacctc ggcttatgcc gaaacgggat cgctgccgcc ggaaacgccc 1200

tttgccaagg atggtgcgct gtccttctat gcggatgcga agaacacgga agcctctgga 1260

aacaaaggcg atgcggtctc catcctcaag gcgctctttg atcgcgtaaa gccgggtgac 1320

tatgtcgggc tgctcgccta tatcaggcgc gaccagcaga cacgggactg ggcacagtcc 1380

gttcgcatgg agcttcggga tgctctgaag gtggcgacag ctgccgaatt cggaccccgg 1440

ttcctgcatt cgacgggcca ggcctacaag ggcggacccg acagtggcgt gttcctgcag 1500

gtcacggcga cggatgcggc agatgtgccg gttccgggcc atggcttcag cttcagtgtc 1560

gtgaaagcgg cacaggcgcg aggcgatttc gaggtgctcg cggcccgtgg tcgtcgtgcc 1620

ctccgcgttc atatcgatgg accgctggag gacgggctga aggctctgga aacggcgatc 1680

cacaaagcac tggcaggagc ataa 1704

<210> 6
<211> 567
<212> PRT
<213> Gluconobacter oxydans DSM 17078

<400> 6

Leu Arg Val Ser Ala Asp Leu Pro Pro Asp Leu Arg Asp Asp Val Ala
1               5                   10                  15

Gly Ala Glu Lys Asp Trp Ser His Ser Gly Gly Leu Arg Arg Ile Trp
            20                  25                  30

Lys Lys Asp Pro Ser Val Trp Thr Asn Gly Pro Glu Ala Gly Trp Leu
        35                  40                  45

Asn Trp Leu Thr Val Val Gln Asp Arg Leu Trp His Ile Glu Glu Leu
    50                  55                  60

Glu Val Leu Ala Arg Asp Val Arg Ser Arg Gly Phe Lys Asp Ile Leu
65                  70                  75                  80

Leu Leu Gly Met Gly Gly Ser Ser Leu Gly Pro Glu Val Leu Ala Glu
                85                  90                  95

Thr Phe Gly Lys Arg Glu Gly Trp Pro Lys Leu His Val Leu Asp Ser
            100                 105                 110

Thr Asp Pro Gln Gln Val Thr Ala Phe Glu Lys Ala Ile Asp Pro Lys
        115                 120                 125

```
Asn Thr Leu Phe Ile Val Ala Ser Lys Ser Gly Gly Thr Leu Glu Pro
    130                 135                 140

Asn Ile Leu Phe Ala His Phe Trp Gln Val Ala Gly Gln Ala Leu Gly
145                 150                 155                 160

Lys Lys Pro Gly Asp Ser Phe Ile Ala Ile Thr Asp Pro Gly Ser His
                165                 170                 175

Met Gln Lys Val Ala Glu Glu Asn Gly Phe Trp Arg Ile Phe Tyr Gly
            180                 185                 190

Asp Pro Lys Ile Gly Gly Arg Tyr Ser Val Leu Ser Asn Phe Gly Leu
        195                 200                 205

Val Pro Ala Ala Ala Ser Gly Ile Asp Val Arg Lys Leu Leu Thr Val
    210                 215                 220

Thr Arg Leu Met Val Glu Ser Cys Asp Gly Ser Ala Pro Pro Ser Ala
225                 230                 235                 240

Asn Pro Gly Ala Val Leu Gly Leu Ile Leu Gly His Ala Ala His Cys
                245                 250                 255

Phe Asn Arg Asp Lys Val Thr Ile Leu Ala Ser Glu Gly Ile Ala Ser
            260                 265                 270

Leu Gly Ala Trp Leu Glu Gln Leu Ile Ala Glu Ser Thr Gly Lys Lys
        275                 280                 285

Gly Leu Gly Leu Ile Pro Ile Asp Glu Glu Glu Leu Gly Thr Pro Asp
    290                 295                 300

Val Tyr Gly Thr Asp Arg Val Phe Val Asp Leu Leu Leu Gly Asp Asp
305                 310                 315                 320

Val Pro Asp Ser Arg Leu Gly Ala Leu Arg Ala Ala Gly Ala Pro Val
                325                 330                 335

Val Thr Ile Arg Leu Ala Asp Lys Ala Gln Ile Gly Gln Ala Phe Phe
            340                 345                 350

Leu Phe Glu Phe Ala Thr Ala Val Ala Gly Ala Val Leu Gly Ile Asp
```

355 360 365

Pro Phe Asp Gln Pro Asp Val Glu Phe Ser Lys Leu Glu Thr Arg Lys
370 375 380

Leu Thr Ser Ala Tyr Ala Glu Thr Gly Ser Leu Pro Pro Glu Thr Pro
385 390 395 400

Phe Ala Lys Asp Gly Ala Leu Ser Phe Tyr Ala Asp Ala Lys Asn Thr
405 410 415

Glu Ala Ser Gly Asn Lys Gly Asp Ala Val Ser Ile Leu Lys Ala Leu
420 425 430

Phe Asp Arg Val Lys Pro Gly Asp Tyr Val Gly Leu Leu Ala Tyr Ile
435 440 445

Arg Arg Asp Gln Gln Thr Arg Asp Trp Ala Gln Ser Val Arg Met Glu
450 455 460

Leu Arg Asp Ala Leu Lys Val Ala Thr Ala Ala Glu Phe Gly Pro Arg
465 470 475 480

Phe Leu His Ser Thr Gly Gln Ala Tyr Lys Gly Gly Pro Asp Ser Gly
485 490 495

Val Phe Leu Gln Val Thr Ala Thr Asp Ala Ala Asp Val Pro Val Pro
500 505 510

Gly His Gly Phe Ser Phe Ser Val Val Lys Ala Ala Gln Ala Arg Gly
515 520 525

Asp Phe Glu Val Leu Ala Ala Arg Gly Arg Arg Ala Leu Arg Val His
530 535 540

Ile Asp Gly Pro Leu Glu Asp Gly Leu Lys Ala Leu Glu Thr Ala Ile
545 550 555 560

His Lys Ala Leu Ala Gly Ala
565

<210> 7
<211> 32
<212> DNA
<213> Artificial

<220>
<223> PCR primer

<400> 7
taagcattta aatctgcggg ttagcgcgga tc 32

<210> 8
<211> 35
<212> DNA
<213> Artificial

<220>
<223> PCR primer

<400> 8
taagcattta aatttatgct cctgccagtg ctttg 35

<210> 9
<211> 2874
<212> DNA
<213> Gluconobacter oxydans DSM 2343

<400> 9
gtggcagaca caatctccaa caccggtctg aacgaagtcg gcagcgtgct gcgcgacctt 60

gaaaaatacg gtcagtctcc ctggctggac ttcatccagc gcagcttcac cgaggatggc 120

agcctcagca agctggtgga agaagacggc ctgcggggtg tgacctccaa cccggccatc 180

ttcgagaagg ccatcgggca cggcacggaa tacgatccgc agatccgcaa gctgctcgaa 240

ggcgagggcc tgtctcccgg cgagctctac gagcatctcg ccatcgacga tatccgctcg 300

gcctgtgcgg tgctggatcc ggtgttcgaa aagaccaagg ggctggacgg ctatgtcagc 360

ctcgaggtct cgccctatct ggccttcgat gcgcacggca ccatcacgga agcgcgccgc 420

ctgtggaagg cggtcgggcg cccgaacctg atgatcaaga ttccgggtac ggttgaaggc 480

acgggtgcga tccgcgaagc cattgcggac ggcatcaatg tcaacgtgac gctgctgttc 540

tcgctggacg cgtacaaagc cgttctggaa gcctatattt ccggtcttga ggcccgtgtg 600

gcccgtggcg aggaaatctc gcgcatctcc agcgtggcgt cgttcttcgt cagccgcatc 660

gatgccaaga tcgacgcgga aatcgaccgc cgcgttgcag cgggtgatcc ggaagccgaa 720

gcgctgaagg ccatccgggg caaggtcgcg atcgccaatg ccaagatggc gtatcagtac 780

tatctggaag tccgcaacag cccgcgctgg aaggcgctgg ccgagaaggg tgcaaacccg 840

cagcgtctgc tgtgggccag cactggcacc aaggacaagg cctattccga tgtcctgtat 900

gtcgaagaac tgatcggtcc cgagacggtt aacaccatcc cgcccgcaac gttcgacgcc 960

tatcgcgatc acggcaagtc ccgtgcgagc ctgacggaga atgtcgatca ggcgcgtcat 1020

gtgatggccg aggccgagcg ccttggactg ggtctggacg ggattacggc caagctggtc 1080

aaagatggtt gtgcggcatt cagcgtctcg ttcgacacgc tgctgacggc ggttgccacc 1140

aagcgcgagg ccgtgctggg cgatcgtctc ctgcgggtca gcgccgacct tccgcccgat 1200

ctgcgtgatg acgttgcagc ggccgaaaag gactggagcc atagcggtgg gctgcgccgc 1260

atctggaaga aggacccgtc cgtctggacg aatggtccgg aagccgggtg gctgaactgg 1320

ctgacggtcg tgcaggatcg tctgtggcat atagaggagc tggaagttct ggctcgcgat 1380

gtccgcagtc gcggcttcaa ggacatcctg cttctgggca tgggcggttc gagccttggt 1440

cccgaggtgc tcgccgagac gttcggaaag cgcgaaggct ggccgaagct gcatgtgctc 1500

gacagcacgg acccgcagca ggttacggcg ttcgaaaagg ccatcgatcc gaagaacacg 1560

ctgttcatcg tggccagcaa gtccggcggc acgctggagc cgaacatcct gttcgcgcat 1620

ttctggcagg ttgcagggca ggctctgggc aagaagccgg gtgacagctt catcgccatc 1680

accgatccgg gctcgcacat gcagaaggtg gccgaggaaa acggcttctg gcgcattttc 1740

tacggcgatc cgaaaattgg tggccgctat tcggttctgt ccaacttcgg tctggtcccg 1800

gcagctgcga gcggcattga tgtccgcaag ctgctgacgg tcacgcgcct gatggtggaa 1860

tcctgtgacg gaagcgcgcc gccttccgcc aatccgggtg cggttctggg cctgatcctg 1920

ggacatgccg cgcactgctt taatcgtgac aaggtcacga tcctggcgtc cgaaggcatt 1980

gcctcgcttg ggcctggct ggagcagttg atcgccgaga gcacgggtaa gaaaggtctg 2040

ggcctcatcc ccatcgatga ggaagagctt ggaacgccgg atgtctacgg aacggaccgt 2100

gtcttcgtgg atctgctgct gggcgacgat gttcccgaca gccgtcttgg ggccctgcgt 2160

gctgctggtg caccggtggt caccatccgt ctggcggaca aggcccagat cggacaggcg 2220

ttcttcctgt tcgagttcgc caccgccgtg gcgggtgcgg ttctggggat cgatccgttc 2280

gatcagccgg atgtggagtt cagcaagctt gagacgcgca aactgacttc ggcctatgcc 2340

gagacgggat cgctgccgcc ggaaacgccc ttcgcaaagg atggtccgct gtccttctat 2400

gcggatgcga agaacacgga agcctctgga aacaaaggcg acgcggtctc catcctcaag 2460

gcgctctttg atcgcgtaaa gccgggtgac tatgtcgggc tgctcgccta tatcaggcgc 2520

gaccagcaga cacgggactg ggcacagtcc gttcgcatgg agcttcggga tgctctgaag 2580

gtggcaacag ctgccgaatt cggaccccgg ttcctgcatt cgacgggcca ggcctacaag 2640

ggcggacccg acagtggcgt gttcctgcag gttacggcga cggatgcggc ggatgtgccg 2700

gttccgggcc atggcttcag cttcagtgtc gtgaaagcgg cacaggcgcg aggcgatttc 2760

gaggtgctcg cggcccgtgg tcgtcgtgcc ctccgcgttc atatcgatgg accgctggag 2820

gacgggctga aggctctgga aacggcgatc cacaaagcac tggcaggagc ataa 2874

<210> 10
<211> 957
<212> PRT
<213> Gluconobacter oxydans DSM 2343

<400> 10

```
Val Ala Asp Thr Ile Ser Asn Thr Gly Leu Asn Glu Val Gly Ser Val
1               5                   10                  15

Leu Arg Asp Leu Glu Lys Tyr Gly Gln Ser Pro Trp Leu Asp Phe Ile
            20                  25                  30

Gln Arg Ser Phe Thr Glu Asp Gly Ser Leu Ser Lys Leu Val Glu Glu
        35                  40                  45

Asp Gly Leu Arg Gly Val Thr Ser Asn Pro Ala Ile Phe Glu Lys Ala
    50                  55                  60

Ile Gly His Gly Thr Glu Tyr Asp Pro Gln Ile Arg Lys Leu Leu Glu
65                  70                  75                  80

Gly Glu Gly Leu Ser Pro Gly Glu Leu Tyr Glu His Leu Ala Ile Asp
                85                  90                  95

Asp Ile Arg Ser Ala Cys Ala Val Leu Asp Pro Val Phe Glu Lys Thr
            100                 105                 110

Lys Gly Leu Asp Gly Tyr Val Ser Leu Glu Val Ser Pro Tyr Leu Ala
        115                 120                 125

Phe Asp Ala His Gly Thr Ile Thr Glu Ala Arg Arg Leu Trp Lys Ala
    130                 135                 140

Val Gly Arg Pro Asn Leu Met Ile Lys Ile Pro Gly Thr Val Glu Gly
145                 150                 155                 160

Thr Gly Ala Ile Arg Glu Ala Ile Ala Asp Gly Ile Asn Val Asn Val
                165                 170                 175

Thr Leu Leu Phe Ser Leu Asp Ala Tyr Lys Ala Val Leu Glu Ala Tyr
            180                 185                 190
```

```
Ile Ser Gly Leu Glu Ala Arg Val Ala Arg Gly Glu Glu Ile Ser Arg
        195                 200                 205

Ile Ser Ser Val Ala Ser Phe Phe Val Ser Arg Ile Asp Ala Lys Ile
    210                 215                 220

Asp Ala Glu Ile Asp Arg Arg Val Ala Ala Gly Asp Pro Glu Ala Glu
225                 230                 235                 240

Ala Leu Lys Ala Ile Arg Gly Lys Val Ala Ile Ala Asn Ala Lys Met
                245                 250                 255

Ala Tyr Gln Tyr Tyr Leu Glu Val Arg Asn Ser Pro Arg Trp Lys Ala
            260                 265                 270

Leu Ala Glu Lys Gly Ala Asn Pro Gln Arg Leu Leu Trp Ala Ser Thr
        275                 280                 285

Gly Thr Lys Asp Lys Ala Tyr Ser Asp Val Leu Tyr Val Glu Glu Leu
    290                 295                 300

Ile Gly Pro Glu Thr Val Asn Thr Ile Pro Pro Ala Thr Phe Asp Ala
305                 310                 315                 320

Tyr Arg Asp His Gly Lys Ser Arg Ala Ser Leu Thr Glu Asn Val Asp
                325                 330                 335

Gln Ala Arg His Val Met Ala Glu Ala Glu Arg Leu Gly Leu Gly Leu
            340                 345                 350

Asp Gly Ile Thr Ala Lys Leu Val Lys Asp Gly Cys Ala Ala Phe Ser
        355                 360                 365

Val Ser Phe Asp Thr Leu Leu Thr Ala Val Ala Thr Lys Arg Glu Ala
    370                 375                 380

Val Leu Gly Asp Arg Leu Leu Arg Val Ser Ala Asp Leu Pro Pro Asp
385                 390                 395                 400

Leu Arg Asp Asp Val Ala Ala Ala Glu Lys Asp Trp Ser His Ser Gly
                405                 410                 415

Gly Leu Arg Arg Ile Trp Lys Lys Asp Pro Ser Val Trp Thr Asn Gly
            420                 425                 430
```

```
Pro Glu Ala Gly Trp Leu Asn Trp Leu Thr Val Val Gln Asp Arg Leu
            435                 440                 445

Trp His Ile Glu Glu Leu Glu Val Leu Ala Arg Asp Val Arg Ser Arg
        450                 455                 460

Gly Phe Lys Asp Ile Leu Leu Leu Gly Met Gly Gly Ser Ser Leu Gly
465                 470                 475                 480

Pro Glu Val Leu Ala Glu Thr Phe Gly Lys Arg Glu Gly Trp Pro Lys
                485                 490                 495

Leu His Val Leu Asp Ser Thr Asp Pro Gln Gln Val Thr Ala Phe Glu
            500                 505                 510

Lys Ala Ile Asp Pro Lys Asn Thr Leu Phe Ile Val Ala Ser Lys Ser
        515                 520                 525

Gly Gly Thr Leu Glu Pro Asn Ile Leu Phe Ala His Phe Trp Gln Val
    530                 535                 540

Ala Gly Gln Ala Leu Gly Lys Lys Pro Gly Asp Ser Phe Ile Ala Ile
545                 550                 555                 560

Thr Asp Pro Gly Ser His Met Gln Lys Val Ala Glu Glu Asn Gly Phe
                565                 570                 575

Trp Arg Ile Phe Tyr Gly Asp Pro Lys Ile Gly Gly Arg Tyr Ser Val
            580                 585                 590

Leu Ser Asn Phe Gly Leu Val Pro Ala Ala Ala Ser Gly Ile Asp Val
        595                 600                 605

Arg Lys Leu Leu Thr Val Thr Arg Leu Met Val Glu Ser Cys Asp Gly
    610                 615                 620

Ser Ala Pro Pro Ser Ala Asn Pro Gly Ala Val Leu Gly Leu Ile Leu
625                 630                 635                 640

Gly His Ala Ala His Cys Phe Asn Arg Asp Lys Val Thr Ile Leu Ala
                645                 650                 655

Ser Glu Gly Ile Ala Ser Leu Gly Ala Trp Leu Glu Gln Leu Ile Ala
```

```
                660                 665                 670

Glu Ser Thr Gly Lys Lys Gly Leu Gly Leu Ile Pro Ile Asp Glu Glu
            675                 680                 685

Glu Leu Gly Thr Pro Asp Val Tyr Gly Thr Asp Arg Val Phe Val Asp
        690                 695                 700

Leu Leu Leu Gly Asp Asp Val Pro Asp Ser Arg Leu Gly Ala Leu Arg
705                 710                 715                 720

Ala Ala Gly Ala Pro Val Val Thr Ile Arg Leu Ala Asp Lys Ala Gln
                725                 730                 735

Ile Gly Gln Ala Phe Phe Leu Phe Glu Phe Ala Thr Ala Val Ala Gly
            740                 745                 750

Ala Val Leu Gly Ile Asp Pro Phe Asp Gln Pro Asp Val Glu Phe Ser
        755                 760                 765

Lys Leu Glu Thr Arg Lys Leu Thr Ser Ala Tyr Ala Glu Thr Gly Ser
    770                 775                 780

Leu Pro Pro Glu Thr Pro Phe Ala Lys Asp Gly Pro Leu Ser Phe Tyr
785                 790                 795                 800

Ala Asp Ala Lys Asn Thr Glu Ala Ser Gly Asn Lys Gly Asp Ala Val
                805                 810                 815

Ser Ile Leu Lys Ala Leu Phe Asp Arg Val Lys Pro Gly Asp Tyr Val
            820                 825                 830

Gly Leu Leu Ala Tyr Ile Arg Arg Asp Gln Gln Thr Arg Asp Trp Ala
        835                 840                 845

Gln Ser Val Arg Met Glu Leu Arg Asp Ala Leu Lys Val Ala Thr Ala
    850                 855                 860

Ala Glu Phe Gly Pro Arg Phe Leu His Ser Thr Gly Gln Ala Tyr Lys
865                 870                 875                 880

Gly Gly Pro Asp Ser Gly Val Phe Leu Gln Val Thr Ala Thr Asp Ala
                885                 890                 895
```

Ala Asp Val Pro Val Pro Gly His Gly Phe Ser Phe Ser Val Val Lys
900 905 910

Ala Ala Gln Ala Arg Gly Asp Phe Glu Val Leu Ala Ala Arg Gly Arg
915 920 925

Arg Ala Leu Arg Val His Ile Asp Gly Pro Leu Glu Asp Gly Leu Lys
930 935 940

Ala Leu Glu Thr Ala Ile His Lys Ala Leu Ala Gly Ala
945 950 955

<210> 11
<211> 24
<212> DNA
<213> Artificial

<220>
<223> PCR primer

<400> 11
gtggcagaca caatctccaa cacc 24

<210> 12
<211> 25
<212> DNA
<213> Artificial

<220>
<223> PCR primer

<400> 12
ttatgctcct gccagtgctt tgtgg 25

<210> 13
<211> 5731
<212> DNA
<213> Artificial

<220>
<223> plasmid

<400> 13
ctagaggctt tcatttaaat cgaatactta tggcagcaga accctgaccg agtggctcac 60

actggtccta ggggttgtca tcattcacat gaagcacggc cagaaggtgc tgtggcagca 120

cccgctggga acggcccgcg ccaatggtcc gtggggcctg ccgaccggtc ttccctggga 180

aatcggtacg ccgaataatg gtggctcggt cgtgacggcc ggtggcgtgg tgttcatcgc 240

ggcagctacg gataaccaga tccgtgccat cgacgagcac accggcaagg tggtctggag 300

cgcggtcctg ccgggcggcg gtcaggctaa cccgatgacc tacgaagcca atggtcatca 360
gtacgtcgcc atcatggcgg gtggtcatca cttcatgatg acgccggtca gcgatcagct 420
ggtggtttac gcactgcctg atcacaaggg ctgagaaagc gggggctgag gccgccgggt 480
gatggaaggg gtggttttcg tgctgcaaat ggcacggaga ccgccccttt gtcgtttccg 540
gggccgtttt ttccagaatt cgatatcaag cttatcgata ccgtcgacct cgaggggggg 600
cccggtaccc agcttttgtt ccctttagtg agggttaatt gcgcgcttgg cgtaatcatg 660
gtcatagctg tttcctgtgt gaaattgtta tccgctcaca attccacaca acatacgagc 720
cggaagcata aagtgtaaag cctggggtgc ctaatgagtg agctaactca cattaattgc 780
gttgcgctca ctgcccgctt tccagtcggg aaacctgtcg tgccagctgc attaatgaat 840
cggccaacgc gcggggagag gcggtttgcg tattgggcgc atgcataaaa actgttgtaa 900
ttcattaagc attctgccga catggaagcc atcacaaacg gcatgatgaa cctgaatcgc 960
cagcggcatc agcaccttgt cgccttgcgt ataatatttg cccatggacg cacaccgtgg 1020
aaacggatga aggcacgaac ccagttgaca taagcctgtt cggttcgtaa actgtaatgc 1080
aagtagcgta tgcgctcacg caactggtcc agaaccttga ccgaacgcag cggtggtaac 1140
ggcgcagtgg cggttttcat ggcttgttat gactgttttt ttgtacagtc tatgcctcgg 1200
gcatccaagc agcaagcgcg ttacgccgtg ggtcgatgtt tgatgttatg gagcagcaac 1260
gatgttacgc agcagcaacg atgttacgca gcagggcagt cgccctaaaa caaagttagg 1320
tggctcaagt atgggcatca ttcgcacatg taggctcggc cctgaccaag tcaaatccat 1380
gcgggctgct cttgatcttt tcggtcgtga gttcggagac gtagccacct actcccaaca 1440
tcagccggac tccgattacc tcgggaactt gctccgtagt aagacattca tcgcgcttgc 1500
tgccttcgac caagaagcgg ttgttggcgc tctcgcggct tacgttctgc ccaggtttga 1560
gcagccgcgt agtgagatct atatctatga tctcgcagtc tccggcgagc accggaggca 1620
gggcattgcc accgcgctca tcaatctcct caagcatgag gccaacgcgc ttggtgctta 1680
tgtgatctac gtgcaagcag attacggtga cgatcccgca gtggctctct atacaaagtt 1740
gggcatacgg gaagaagtga tgcactttga tatcgaccca agtaccgcca cctaacaatt 1800
cgttcaagcc gagatcggct tcccggccgc ggagttgttc ggtaaattgt cacaacgccg 1860
ccaggtggca cttttcgggg aaatgtgcgc gcccgcgttc ctgctggcgc tgggcctgtt 1920
tctggcgctg gacttcccgc tgttccgtca gcagcttttc gcccacggcc ttgatgatcg 1980
cggcggcctt ggcctgcata tcccgattca acggccccag ggcgtccaga acgggcttca 2040
ggcgctcccg aaggtctcgg gccgtctctt gggcttgatc ggccttcttg cgcatctcac 2100

gcgctcctgc ggcggcctgt agggcaggct catacccctg ccgaaccgct tttgtcagcc 2160

ggtcggccac ggcttccggc gtctcaacgc gctttgagat tcccagcttt tcggccaatc 2220

cctgcggtgc ataggcgcgt ggctcgaccg cttgcgggct gatggtgacg tggcccactg 2280

gtggccgctc cagggcctcg tagaacgcct gaatgcgcgt gtgacgtgcc ttgctgccct 2340

cgatgccccg ttgcagccct agatcggcca cagcggccgc aaacgtggtc tggtcgcggg 2400

tcatctgcgc tttgttgccg atgaactcct tggccgacag cctgccgtcc tgcgtcagcg 2460

gcaccacgaa cgcggtcatg tgcgggctgg tttcgtcacg gtggatgctg gccgtcacga 2520

tgcgatccgc cccgtacttg tccgccagcc acttgtgcgc cttctcgaag aacgccgcct 2580

gctgttcttg gctggccgac ttccaccatt ccgggctggc cgtcatgacg tactcgaccg 2640

ccaacacagc gtccttgcgc cgcttctctg gcagcaactc gcgcagtcgg cccatcgctt 2700

catcggtgct gctggccgcc cagtgctcgt tctctggcgt cctgctggcg tcagcgttgg 2760

gcgtctcgcg ctcgcggtag gcgtgcttga gactggccgc cacgttgccc attttcgcca 2820

gcttcttgca tcgcatgatc gcgtatgccg ccatgcctgc ccctcccttt tggtgtccaa 2880

ccggctcgac gggggcagcg caaggcggtg cctccggcgg gccactcaat gcttgagtat 2940

actcactaga ctttgcttcg caaagtcgtg accgcctacg gcggctgcgg cgccctacgg 3000

gcttgctctc cgggcttcgc cctgcgcggt cgctgcgctc ccttgccagc ccgtggatat 3060

gtggacgatg gccgcgagcg gccaccggct ggctcgcttc gctcggcccg tggacaaccc 3120

tgctggacaa gctgatggac aggctgcgcc tgcccacgag cttgaccaca gggattgccc 3180

accggctacc cagccttcga ccacataccc accggctcca actgcgcggc ctgcggcctt 3240

gccccatcaa ttttttaat tttctctggg gaaaagcctc cggcctgcgg cctgcgcgct 3300

tcgcttgccg gttggacacc aagtggaagg cgggtcaagg ctcgcgcagc gaccgcgcag 3360

cggcttggcc ttgacgcgcc tggaacgacc caagcctatg cgagtggggg cagtcgaagg 3420

cgaagcccgc ccgcctgccc cccgagcctc acggcggcga gtgcgggggt tccaaggggg 3480

cagcgccacc ttgggcaagg ccgaaggccg cgcagtcgat caacaagccc cggaggggcc 3540

actttttgcc ggagggggag ccgcgccgaa ggcgtggggg aaccccgcag ggtgcccttt 3600

ctttgggcac caaagaacta gatatagggc gaaatgcgaa agacttaaaa atcaacaact 3660

taaaaaaggg gggtacgcaa cagctcattg cggcaccccc cgcaatagct cattgcgtag 3720

gttaaagaaa atctgtaatt gactgccact tttacgcaac gcataattgt tgtcgcgctg 3780

ccgaaaagtt gcagctgatt gcgcatggtg ccgcaaccgt gcggcaccct accgcatgga 3840

```
gataagcatg gccacgcagt ccagagaaat cggcattcaa gccaagaaca agcccggtca   3900
ctgggtgcaa acggaacgca aagcgcatga ggcgtgggcc gggcttattg cgaggaaacc   3960
cacggcggca atgctgctgc atcacctcgt ggcgcagatg ggccaccaga acgccgtggt   4020
ggtcagccag aagacacttt ccaagctcat cggacgttct ttgcggacgg tccaatacgc   4080
agtcaaggac ttggtggccg agcgctggat ctccgtcgtg aagctcaacg gccccggcac   4140
cgtgtcggcc tacgtggtca atgaccgcgt ggcgtggggc cagccccgcg accagttgcg   4200
cctgtcggtg ttcagtgccg ccgtggtggt tgatcacgac gaccaggacg aatcgctgtt   4260
ggggcatggc gacctgcgcc gcatcccgac cctgtatccg ggcgagcagc aactaccgac   4320
cggccccggc gaggagccgc ccagccagcc cggcattccg ggcatggaac cagacctgcc   4380
agccttgacc gaaacggagg aatgggaacg gcgcgggcag cagcgcctgc cgatgcccga   4440
tgagccgtgt ttctggacg atggcgagcc gttggagccg ccgacacggg tcacgctgcc   4500
gcgccggtag cacttgggtt gcgcagcaac ccgtaagtgc gctgttccag actatcggct   4560
gtagccgcct cgccgcccta taccttgtct gcctccccgc gttgcgtcgc ggtgcatgga   4620
gccgggccac ctcgacctga atggaagccg gcggcacctc gctaacggat tcaccgtttt   4680
tatcaggctc tgggaggcag aataaatgat catatcgtca attattacct ccacggggag   4740
agcctgagca aactggcctc aggcatttga gaagcacacg gtcacactgc ttccggtagt   4800
caataaaccg gtaaaccagc aatagacata agcggctatt taacgaccct gccctgaacc   4860
gacgaccggg tcgaatttgc tttcgaattt ctgccattca tccgcttatt atcacttatt   4920
caggcgtagc accaggcgtt taagggcacc aataactgcc ttaaaaaaat tacgccccgc   4980
cctgccactc atcgcagtcg gcctattggt taaaaaatga gctgatttaa caaaaattta   5040
acgcgaattt taacaaaata ttaacgctta caatttccat tcgccattca ggctgcgcaa   5100
ctgttgggaa gggcgatcgg tgcgggcctc ttcgctatta cgccagctgg cgaaaggggg   5160
atgtgctgca aggcgattaa gttgggtaac gccagggttt tcccagtcac gcaattgtca   5220
tatgacgact agtgagcgcg cgtaatacga ctcactatag ggcgaattgg agctccactg   5280
ccgaaggcat cgtttacggt gctctcgaag tcatgcgccg tcgcggcggc acgactgcag   5340
atccggtggc catgttccac tcggctctgg ataacgtgaa gcctgcggtt gaagtccgct   5400
cgcgtcgcgt cggtggcgca acctatcagg ttccggttga agtccgcgcc gagcgccgtc   5460
aggctctcgc gatccgctgg ctgatcgatg cctcccgcaa gcgtggcgag aacacgatgc   5520
aggagcgtct gtccaacgaa ctgatggatg cggtcaacaa ccgtggctcc gctgtcaaga   5580
agcgcgaaga cacgcaccgc atggctgaag ccaacaaggc attcagccac tatcgctggt   5640
```

aatcagaacc ggttaaaggg cttccggcag tgtgtcgatc tcaggatcgt gcactgactg 5700

gtttgaactt tcagtcttac tcccgttcca t 5731

<210> 14
<211> 8605
<212> DNA
<213> Artificial

<220>
<223> plasmid

<400> 14
ctagaggctt tcatttgtgg cagacacaat ctccaacacc ggtctgaacg aagtcggcag 60

cgtgctgcgc gaccttgaaa aatacggtca gtctccctgg ctggacttca tccagcgcag 120

cttcaccgag gatggcagcc tcagcaagct ggtggaagaa gacggcctgc ggggtgtgac 180

ctccaacccg gccatcttcg agaaggccat cgggcacggc acggaatacg atccgcagat 240

ccgcaagctg ctcgaaggcg agggcctgtc tcccggcgag ctctacgagc atctcgccat 300

cgacgatatc cgctcggcct gtgcggtgct ggatccggtg ttcgaaaaga ccaaggggct 360

ggacggctat gtcagcctcg aggtctcgcc ctatctggcc ttcgatgcgc acggcaccat 420

cacggaagcg cgccgcctgt ggaaggcggt cgggcgcccg aacctgatga tcaagattcc 480

gggtacggtt gaaggcacgg gtgcgatccg cgaagccatt gcggacggca tcaatgtcaa 540

cgtgacgctg ctgttctcgc tggacgcgta caaagccgtt ctggaagcct atatttccgg 600

tcttgaggcc cgtgtggccc gtggcgagga aatctcgcgc atctccagcg tggcgtcgtt 660

cttcgtcagc cgcatcgatg ccaagatcga cgcggaaatc gaccgccgcg ttgcagcggg 720

tgatccggaa gccgaagcgc tgaaggccat ccggggcaag gtcgcgatcg ccaatgccaa 780

gatggcgtat cagtactatc tggaagtccg caacagcccg cgctggaagg cgctggccga 840

gaagggtgca aacccgcagc gtctgctgtg ggccagcact ggcaccaagg acaaggccta 900

ttccgatgtc ctgtatgtcg aagaactgat cggtcccgag acggttaaca ccatcccgcc 960

cgcaacgttc gacgcctatc gcgatcacgg caagtcccgt gcgagcctga cggagaatgt 1020

cgatcaggcg cgtcatgtga tggccgaggc cgagcgcctt ggactgggtc tggacgggat 1080

tacggccaag ctggtcaaag atggttgtgc ggcattcagc gtctcgttcg acacgctgct 1140

gacggcggtt gccaccaagc gcgaggccgt gctgggcgat cgtctcctgc gggtcagcgc 1200

cgaccttccg cccgatctgc gtgatgacgt tgcagcggcc gaaaaggact ggagccatag 1260

cggtgggctg cgccgcatct ggaagaagga cccgtccgtc tggacgaatg gtccggaagc 1320

cgggtggctg aactggctga cggtcgtgca ggatcgtctg tggcatatag aggagctgga 1380

agttctggct cgcgatgtcc gcagtcgcgg cttcaaggac atcctgcttc tgggcatggg 1440

cggttcgagc cttggtcccg aggtgctcgc cgagacgttc ggaaagcgcg aaggctggcc 1500

gaagctgcat gtgctcgaca gcacggaccc gcagcaggtt acggcgttcg aaaaggccat 1560

cgatccgaag aacacgctgt tcatcgtggc cagcaagtcc ggcggcacgc tggagccgaa 1620

catcctgttc gcgcatttct ggcaggttgc agggcaggct ctgggcaaga agccgggtga 1680

cagcttcatc gccatcaccg atccgggctc gcacatgcag aaggtggccg aggaaaacgg 1740

cttctggcgc attttctacg gcgatccgaa aattggtggc cgctattcgg ttctgtccaa 1800

cttcggtctg gtcccggcag ctgcgagcgg cattgatgtc cgcaagctgc tgacggtcac 1860

gcgcctgatg gtggaatcct gtgacggaag cgcgccgcct tccgccaatc cgggtgcggt 1920

tctgggcctg atcctgggac atgccgcgca ctgctttaat cgtgacaagg tcacgatcct 1980

ggcgtccgaa ggcattgcct cgcttggggc ctggctggag cagttgatcg ccgagagcac 2040

gggtaagaaa ggtctgggcc tcatccccat cgatgaggaa gagcttggaa cgccggatgt 2100

ctacggaacg gaccgtgtct tcgtggatct gctgctgggc gacgatgttc ccgacagccg 2160

tcttggggcc ctgcgtgctg ctggtgcacc ggtggtcacc atccgtctgg cggacaaggc 2220

ccagatcgga caggcgttct tcctgttcga gttcgccacc gccgtggcgg gtgcggttct 2280

ggggatcgat ccgttcgatc agccggatgt ggagttcagc aagcttgaga cgcgcaaact 2340

gacttcggcc tatgccgaga cgggatcgct gccgccggaa acgcccttcg caaaggatgg 2400

tccgctgtcc ttctatgcgg atgcgaagaa cacggaagcc tctggaaaca aaggcgacgc 2460

ggtctccatc ctcaaggcgc tctttgatcg cgtaaagccg ggtgactatg tcgggctgct 2520

cgcctatatc aggcgcgacc agcagacacg ggactgggca cagtccgttc gcatggagct 2580

tcgggatgct ctgaaggtgg caacagctgc cgaattcgga ccccggttcc tgcattcgac 2640

gggccaggcc tacaagggcg gacccgacag tggcgtgttc ctgcaggtta cggcgacgga 2700

tgcggcggat gtgccggttc cgggccatgg cttcagcttc agtgtcgtga aagcggcaca 2760

ggcgcgaggc gatttcgagg tgctcgcggc ccgtggtcgt cgtgccctcc gcgttcatat 2820

cgatggaccg ctggaggacg ggctgaaggc tctggaaacg gcgatccaca aagcactggc 2880

aggagcataa aaatcgaata cttatggcag cagaaccctg accgagtggc tcacactggt 2940

cctaggggtt gtcatcattc acatgaagca cggccagaag gtgctgtggc agcacccgct 3000

gggaacggcc cgcgccaatg gtccgtgggg cctgccgacc ggtcttccct gggaaatcgg 3060

tacgccgaat aatggtggct cggtcgtgac ggccggtggc gtggtgttca tcgcggcagc 3120

tacggataac cagatccgtg ccatcgacga gcacaccggc aaggtggtct ggagcgcggt 3180
cctgccgggc ggcggtcagg ctaacccgat gacctacgaa gccaatggtc atcagtacgt 3240
cgccatcatg gcgggtggtc atcacttcat gatgacgccg gtcagcgatc agctggtggt 3300
ttacgcactg cctgatcaca agggctgaga aagcgggggc tgaggccgcc gggtgatgga 3360
aggggtggtt ttcgtgctgc aaatggcacg gagaccgccc ctttgtcgtt tccggggccg 3420
ttttttccag aattcgatat caagcttatc gataccgtcg acctcgaggg ggggcccggt 3480
acccagcttt tgttcccttt agtgagggtt aattgcgcgc ttggcgtaat catggtcata 3540
gctgtttcct gtgtgaaatt gttatccgct cacaattcca cacaacatac gagccggaag 3600
cataaagtgt aaagcctggg gtgcctaatg agtgagctaa ctcacattaa ttgcgttgcg 3660
ctcactgccc gctttccagt cgggaaacct gtcgtgccag ctgcattaat gaatcggcca 3720
acgcgcgggg agaggcggtt tgcgtattgg gcgcatgcat aaaaactgtt gtaattcatt 3780
aagcattctg ccgacatgga agccatcaca aacggcatga tgaacctgaa tcgccagcgg 3840
catcagcacc ttgtcgcctt gcgtataata tttgcccatg gacgcacacc gtggaaacgg 3900
atgaaggcac gaacccagtt gacataagcc tgttcggttc gtaaactgta atgcaagtag 3960
cgtatgcgct cacgcaactg gtccagaacc ttgaccgaac gcagcggtgg taacggcgca 4020
gtggcggttt tcatggcttg ttatgactgt ttttttgtac agtctatgcc tcgggcatcc 4080
aagcagcaag cgcgttacgc cgtgggtcga tgtttgatgt tatggagcag caacgatgtt 4140
acgcagcagc aacgatgtta cgcagcaggg cagtcgccct aaaacaaagt taggtggctc 4200
aagtatgggc atcattcgca catgtaggct cggccctgac caagtcaaat ccatgcgggc 4260
tgctcttgat cttttcggtc gtgagttcgg agacgtagcc acctactccc aacatcagcc 4320
ggactccgat tacctcggga acttgctccg tagtaagaca ttcatcgcgc ttgctgcctt 4380
cgaccaagaa gcggttgttg gcgctctcgc ggcttacgtt ctgcccaggt ttgagcagcc 4440
gcgtagtgag atctatatct atgatctcgc agtctccggc gagcaccgga ggcagggcat 4500
tgccaccgcg ctcatcaatc tcctcaagca tgaggccaac gcgcttggtg cttatgtgat 4560
ctacgtgcaa gcagattacg gtgacgatcc cgcagtggct ctctatacaa agttgggcat 4620
acgggaagaa gtgatgcact ttgatatcga cccaagtacc gccacctaac aattcgttca 4680
agccgagatc ggcttcccgg ccgcggagtt gttcggtaaa ttgtcacaac gccgccaggt 4740
ggcacttttc ggggaaatgt gcgcgcccgc gttcctgctg gcgctgggcc tgtttctggc 4800
gctggacttc ccgctgttcc gtcagcagct tttcgcccac ggccttgatg atcgcggcgg 4860

ccttggcctg catatcccga ttcaacggcc ccagggcgtc cagaacgggc ttcaggcgct 4920
cccgaaggtc tcgggccgtc tcttgggctt gatcggcctt cttgcgcatc tcacgcgctc 4980
ctgcggcggc ctgtagggca ggctcatacc cctgccgaac cgcttttgtc agccggtcgg 5040
ccacggcttc cggcgtctca acgcgctttg agattcccag cttttcggcc aatccctgcg 5100
gtgcataggc gcgtggctcg accgcttgcg ggctgatggt gacgtggccc actggtggcc 5160
gctccagggc ctcgtagaac gcctgaatgc gcgtgtgacg tgccttgctg ccctcgatgc 5220
cccgttgcag ccctagatcg gccacagcgg ccgcaaacgt ggtctggtcg cgggtcatct 5280
gcgctttgtt gccgatgaac tccttggccg acagcctgcc gtcctgcgtc agcggcacca 5340
cgaacgcggt catgtgcggg ctggtttcgt cacggtggat gctggccgtc acgatgcgat 5400
ccgccccgta cttgtccgcc agccacttgt gcgccttctc gaagaacgcc gcctgctgtt 5460
cttggctggc cgacttccac cattccgggc tggccgtcat gacgtactcg accgccaaca 5520
cagcgtcctt gcgccgcttc tctggcagca actcgcgcag tcggcccatc gcttcatcgg 5580
tgctgctggc cgcccagtgc tcgttctctg gcgtcctgct ggcgtcagcg ttgggcgtct 5640
cgcgctcgcg gtaggcgtgc ttgagactgg ccgccacgtt gcccattttc gccagcttct 5700
tgcatcgcat gatcgcgtat gccgccatgc ctgcccctcc cttttggtgt ccaaccggct 5760
cgacgggggc agcgcaaggc ggtgcctccg gcgggccact caatgcttga gtatactcac 5820
tagactttgc ttcgcaaagt cgtgaccgcc tacggcggct gcggcgccct acgggcttgc 5880
tctccgggct tcgccctgcg cggtcgctgc gctcccttgc cagcccgtgg atatgtggac 5940
gatggccgcg agcggccacc ggctggctcg cttcgctcgg cccgtggaca accctgctgg 6000
acaagctgat ggacaggctg cgcctgccca cgagcttgac cacagggatt gcccaccggc 6060
tacccagcct tcgaccacat acccaccggc tccaactgcg cggcctgcgg ccttgcccca 6120
tcaatttttt taattttctc tggggaaaag cctccggcct gcggcctgcg cgcttcgctt 6180
gccggttgga caccaagtgg aaggcgggtc aaggctcgcg cagcgaccgc gcagcggctt 6240
ggccttgacg cgcctggaac gacccaagcc tatgcgagtg gggcagtcg aaggcgaagc 6300
ccgcccgcct gccccccgag cctcacggcg gcgagtgcgg gggttccaag gggcagcgc 6360
caccttgggc aaggccgaag gccgcgcagt cgatcaacaa gccccggagg ggccactttt 6420
tgccggaggg ggagccgcgc cgaaggcgtg ggggaacccc gcaggggtgc ccttctttgg 6480
gcaccaaaga actagatata gggcgaaatg cgaaagactt aaaaatcaac aacttaaaaa 6540
aggggggtac gcaacagctc attgcggcac cccccgcaat agctcattgc gtaggttaaa 6600
gaaaatctgt aattgactgc cacttttacg caacgcataa ttgttgtcgc gctgccgaaa 6660

agttgcagct gattgcgcat ggtgccgcaa ccgtgcggca ccctaccgca tggagataag 6720
catggccacg cagtccagag aaatcggcat tcaagccaag aacaagcccg gtcactgggt 6780
gcaaacggaa cgcaaagcgc atgaggcgtg ggccgggctt attgcgagga aacccacggc 6840
ggcaatgctg ctgcatcacc tcgtggcgca gatgggccac cagaacgccg tggtggtcag 6900
ccagaagaca ctttccaagc tcatcggacg ttctttgcgg acggtccaat acgcagtcaa 6960
ggacttggtg gccgagcgct ggatctccgt cgtgaagctc aacggccccg gcaccgtgtc 7020
ggcctacgtg gtcaatgacc gcgtggcgtg ggccagcccc cgcgaccagt tgcgcctgtc 7080
ggtgttcagt gccgccgtgg tggttgatca cgacgaccag gacgaatcgc tgttggggca 7140
tggcgacctg cgccgcatcc cgaccctgta tccgggcgag cagcaactac cgaccggccc 7200
cggcgaggag ccgcccagcc agcccggcat tccgggcatg gaaccagacc tgccagcctt 7260
gaccgaaacg gaggaatggg aacggcgcgg gcagcagcgc ctgccgatgc ccgatgagcc 7320
gtgttttctg gacgatggcg agccgttgga gccgccgaca cgggtcacgc tgccgcgccg 7380
gtagcacttg ggttgcgcag caacccgtaa gtgcgctgtt ccagactatc ggctgtagcc 7440
gcctcgccgc cctatacctt gtctgcctcc ccgcgttgcg tcgcggtgca tggagccggg 7500
ccacctcgac ctgaatggaa gccggcggca cctcgctaac ggattcaccg tttttatcag 7560
gctctgggag gcagaataaa tgatcatatc gtcaattatt acctccacgg ggagagcctg 7620
agcaaactgg cctcaggcat ttgagaagca cacggtcaca ctgcttccgg tagtcaataa 7680
accggtaaac cagcaataga cataagcggc tatttaacga ccctgccctg aaccgacgac 7740
cgggtcgaat ttgctttcga atttctgcca ttcatccgct tattatcact tattcaggcg 7800
tagcaccagg cgtttaaggg caccaataac tgccttaaaa aaattacgcc ccgccctgcc 7860
actcatcgca gtcggcctat tggttaaaaa atgagctgat ttaacaaaaa tttaacgcga 7920
attttaacaa aatattaacg cttacaattt ccattcgcca ttcaggctgc gcaactgttg 7980
ggaagggcga tcggtgcggg cctcttcgct attacgccag ctggcgaaag ggggatgtgc 8040
tgcaaggcga ttaagttggg taacgccagg gttttcccag tcacgcaatt gtcatatgac 8100
gactagtgag cgcgcgtaat acgactcact atagggcgaa ttggagctcc actgccgaag 8160
gcatcgttta cggtgctctc gaagtcatgc gccgtcgcgg cggcacgact gcagatccgg 8220
tggccatgtt ccactcggct ctggataacg tgaagcctgc ggttgaagtc cgctcgcgtc 8280
gcgtcggtgg cgcaacctat caggttccgg ttgaagtccg cgccgagcgc cgtcaggctc 8340
tcgcgatccg ctggctgatc gatgcctccc gcaagcgtgg cgagaacacg atgcaggagc 8400

gtctgtccaa cgaactgatg gatgcggtca acaaccgtgg ctccgctgtc aagaagcgcg 8460

aagacacgca ccgcatggct gaagccaaca aggcattcag ccactatcgc tggtaatcag 8520

aaccggttaa agggcttccg gcagtgtgtc gatctcagga tcgtgcactg actggtttga 8580

actttcagtc ttactcccgt tccat 8605

<210> 15
<211> 9053
<212> DNA
<213> Artificial

<220>
<223> plasmid

<400> 15
ctcgggccgt ctcttgggct tgatcggcct tcttgcgcat ctcacgcgct cctgcggcgg 60

cctgtagggc aggctcatac ccctgccgaa ccgcttttgt cagccggtcg gccacggctt 120

ccggcgtctc aacgcgcttt gagattccca gcttttcggc caatccctgc ggtgcatagg 180

cgcgtggctc gaccgcttgc gggctgatgg tgacgtggcc cactggtggc cgctccaggg 240

cctcgtagaa cgcctgaatg cgcgtgtgac gtgccttgct gccctcgatg ccccgttgca 300

gccctagatc ggccacagcg gccgcaaacg tggtctggtc gcgggtcatc tgcgctttgt 360

tgccgatgaa ctccttggcc gacagcctgc cgtcctgcgt cagcggcacc acgaacgcgg 420

tcatgtgcgg gctggtttcg tcacggtgga tgctggccgt cacgatgcga tccgccccgt 480

acttgtccgc cagccacttg tgcgccttct cgaagaacgc cgcctgctgt tcttggctgg 540

ccgacttcca ccattccggg ctggccgtca tgacgtactc gaccgccaac acagcgtcct 600

tgcgccgctt ctctggcagc aactcgcgca gtcggcccat cgcttcatcg gtgctgctgg 660

ccgcccagtg ctcgttctct ggcgtcctgc tggcgtcagc gttgggcgtc tcgcgctcgc 720

ggtaggcgtg cttgagactg gccgccacgt tgcccatttt cgccagcttc ttgcatcgca 780

tgatcgcgta tgccgccatg cctgcccctc ccttttggtg tccaaccggc tcgacggggg 840

cagcgcaagg cggtgcctcc ggcgggccac tcaatgcttg agtatactca ctagactttg 900

cttcgcaaag tcgtgaccgc ctacggcggc tgcggcgccc tacgggcttg ctctccgggc 960

ttcgccctgc gcggtcgctg cgctcccttg ccagcccgtg gatatgtgga cgatggccgc 1020

gagcggccac cggctggctc gcttcgctcg gcccgtggac aaccctgctg gacaagctga 1080

tggacaggct gcgcctgccc acgagcttga ccacagggat tgcccaccgg ctacccagcc 1140

ttcgaccaca tacccaccgg ctccaactgc gcggcctgcg gccttgcccc atcaattttt 1200

ttaattttct ctggggaaaa gcctccggcc tgcggcctgc gcgcttcgct tgccggttgg 1260

```
acaccaagtg gaaggcgggt caaggctcgc gcagcgaccg cgcagcggct tggccttgac   1320
gcgcctggaa cgacccaagc ctatgcgagt gggggcagtc gaaggcgaag cccgcccgcc   1380
tgccccccga gcctcacggc ggcgagtgcg ggggttccaa gggggcagcg ccaccttggg   1440
caaggccgaa ggccgcgcag tcgatcaaca agccccggag gggccacttt ttgccggagg   1500
gggagccgcg ccgaaggcgt gggggaaccc cgcaggggtg cccttctttg ggcaccaaag   1560
aactagatat agggcgaaat gcgaaagact taaaaatcaa caacttaaaa aagggggggta   1620
cgcaacagct cattgcggca ccccccgcaa tagctcattg cgtaggttaa agaaaatctg   1680
taattgactg ccacttttac gcaacgcata attgttgtcg cgctgccgaa aagttgcagc   1740
tgattgcgca tggtgccgca accgtgcggc accctaccgc atggagataa gcatggccac   1800
gcagtccaga gaaatcggca ttcaagccaa gaacaagccc ggtcactggg tgcaaacgga   1860
acgcaaagcg catgaggcgt gggccgggct tattgcgagg aaacccacgg cggcaatgct   1920
gctgcatcac ctcgtggcgc agatgggcca ccagaacgcc gtggtggtca gccagaagac   1980
actttccaag ctcatcggac gttctttgcg gacggtccaa tacgcagtca aggacttggt   2040
ggccgagcgc tggatctccg tcgtgaagct caacggcccc ggcaccgtgt cggcctacgt   2100
ggtcaatgac cgcgtggcgt ggggccagcc ccgcgaccag ttgcgcctgt cggtgttcag   2160
tgccgccgtg gtggttgatc acgacgacca ggacgaatcg ctgttggggc atggcgacct   2220
gcgccgcatc ccgaccctgt atccgggcga gcagcaacta ccgaccggcc ccggcgagga   2280
gccgcccagc cagcccggca ttccgggcat ggaaccagac ctgccagcct tgaccgaaac   2340
ggaggaatgg gaacggcgcg ggcagcagcg cctgccgatg cccgatgagc cgtgttttct   2400
ggacgatggc gagccgttgg agccgccgac acgggtcacg ctgccgcgcc ggtagcactt   2460
gggttgcgca gcaacccgta agtgcgctgt tccagactat cggctgtagc cgcctcgccg   2520
ccctatacct tgtctgcctc cccgcgttgc gtcgcggtgc atggagccgg gccacctcga   2580
cctgaatgga agccggcggc acctcgctaa cggattcacc gtttttatca ggctctggga   2640
ggcagaataa atgatcatat cgtcaattat tacctccacg gggagagcct gagcaaactg   2700
gcctcaggca tttgagaagc acacggtcac actgcttccg gtagtcaata aaccggtaaa   2760
ccagcaatag acataagcgg ctatttaacg accctgccct gaaccgacga ccgggtcgaa   2820
tttgctttcg aatttctgcc attcatccgc ttattatcac ttattcaggc gtagcaccag   2880
gcgtttaagg gcaccaataa ctgccttaaa aaaattacgc cccgccctgc cactcatcgc   2940
agtcggccta ttggttaaaa aatgagctga tttaacaaaa atttaacgcg aattttaaca   3000
```

aaatattaac gcttacaatt tccattcgcc attcaggctg cgcaactgtt gggaagggcg 3060

atcggtgcgg gcctcttcgc tattacgcca gctggcgaaa gggggatgtg ctgcaaggcg 3120

attaagttgg gtaacgccag ggttttccca gtcacgacgt tgtaaaacga cggccagtga 3180

gcgcgcgtaa tacgactcac tatagggcga attggagctc caccgcggtg gcggccgctc 3240

tagaactagt gagcgcgcgt aatacgactc actatagggc gaattggagc tccactgccg 3300

aaggcatcgt ttacggtgct ctcgaagtca tgcgccgtcg cggcggcacg actgcagatc 3360

cggtggccat gttccactcg gctctggata acgtgaagcc tgcggttgaa gtccgctcgc 3420

gtcgcgtcgg tggcgcaacc tatcaggttc cggttgaagt ccgcgccgag cgccgtcagg 3480

ctctcgcgat ccgctggctg atcgatgcct cccgcaagcg tggcgagaac acgatgcagg 3540

agcgtctgtc caacgaactg atggatgcgg tcaacaaccg tggctccgct gtcaagaagc 3600

gcgaagacac gcaccgcatg gctgaagcca acaaggcatt cagccactat cgctggtaat 3660

cagaaccggt taaagggctt ccggcagtgt gtcgatctca ggatcgtgca ctgactggtt 3720

tgaactttca gtcttactcc cgttccatct agaggctttc atttgtggca gacacaatct 3780

ccaacaccgg tctgaacgaa gtcggcagcg tgctgcgcga ccttgaaaaa tacggtcagt 3840

ctccctggct ggacttcatc cagcgcagct tcaccgagga tggcagcctc agcaagctgg 3900

tggaagaaga cggcctgcgg ggtgtgacct ccaacccggc catcttcgag aaggccatcg 3960

ggcacggcac ggaatacgat ccgcagatcc gcaagctgct cgaaggcgag ggcctgtctc 4020

ccggcgagct ctacgagcat ctcgccatcg acgatatccg ctcggcctgt gcggtgctgg 4080

atccggtgtt cgaaaagacc aaggggctgg acggctatgt cagcctcgag gtctcgccct 4140

atctggcctt cgatgcgcac ggcaccatca cggaagcgcg ccgcctgtgg aaggcggtcg 4200

ggcgcccgaa cctgatgatc aagattccgg gtacggttga aggcacgggt gcgatccgcg 4260

aagccattgc ggacggcatc aatgtcaacg tgacgctgct gttctcgctg gacgcgtaca 4320

aagccgttct ggaagcctat atttccggtc ttgaggcccg tgtggcccgt ggcgaggaaa 4380

tctcgcgcat ctccagcgtg gcgtcgttct tcgtcagccg catcgatgcc aagatcgacg 4440

cggaaatcga ccgccgcgtt gcagcgggtg atccggaagc cgaagcgctg aaggccatcc 4500

ggggcaaggt cgcgatcgcc aatgccaaga tggcgtatca gtactatctg gaagtccgca 4560

acagcccgcg ctggaaggcg ctggccgaga agggtgcaaa cccgcagcgt ctgctgtggg 4620

ccagcactgg caccaaggac aaggcctatt ccgatgtcct gtatgtcgaa gaactgatcg 4680

gtcccgagac ggttaacacc atcccgcccg caacgttcga cgcctatcgc gatcacggca 4740

agtcccgtgc gagcctgacg gagaatgtcg atcaggcgcg tcatgtgatg gccgaggccg 4800

agcgccttgg actgggtctg gacgggatta cggccaagct ggtcaaagat ggttgtgcgg 4860
cattcagcgt ctcgttcgac acgctgctga cggcggttgc caccaagcgc gaggccgtgc 4920
tgggcgatcg tctcctgcgg gtcagcgccg accttccgcc cgatctgcgt gatgacgttg 4980
cagcggccga aaaggactgg agccatagcg gtgggctgcg ccgcatctgg aagaaggacc 5040
cgtccgtctg gacgaatggt ccggaagccg ggtggctgaa ctggctgacg gtcgtgcagg 5100
atcgtctgtg gcatatagag gagctggaag ttctggctcg cgatgtccgc agtcgcggct 5160
tcaaggacat cctgcttctg ggcatgggcg gttcgagcct tggtcccgag gtgctcgccg 5220
agacgttcgg aaagcgcgaa ggctggccga agctgcatgt gctcgacagc acggacccgc 5280
agcaggttac ggcgttcgaa aaggccatcg atccgaagaa cacgctgttc atcgtggcca 5340
gcaagtccgg cggcacgctg gagccgaaca tcctgttcgc gcatttctgg caggttgcag 5400
ggcaggctct gggcaagaag ccgggtgaca gcttcatcgc catcaccgat ccgggctcgc 5460
acatgcagaa ggtggccgag gaaaacggct tctggcgcat tttctacggc gatccgaaaa 5520
ttggtggccg ctattcggtt ctgtccaact tcggtctggt cccggcagct gcgagcggca 5580
ttgatgtccg caagctgctg acggtcacgc gcctgatggt ggaatcctgt gacggaagcg 5640
cgccgccttc cgccaatccg ggtgcggttc tgggcctgat cctgggacat gccgcgcact 5700
gctttaatcg tgacaaggtc acgatcctgg cgtccgaagg cattgcctcg cttggggcct 5760
ggctggagca gttgatcgcc gagagcacgg gtaagaaagg tctgggcctc atccccatcg 5820
atgaggaaga gcttggaacg ccggatgtct acggaacgga ccgtgtcttc gtggatctgc 5880
tgctgggcga cgatgttccc gacagccgtc ttggggccct gcgtgctgct ggtgcaccgg 5940
tggtcaccat ccgtctggcg gacaaggccc agatcggaca ggcgttcttc ctgttcgagt 6000
tcgccaccgc cgtggcgggt gcggttctgg ggatcgatcc gttcgatcag ccggatgtgg 6060
agttcagcaa gcttgagacg cgcaaactga cttcggccta tgccgagacg ggatcgctgc 6120
cgccggaaac gcccttcgca aaggatggtc cgctgtcctt ctatgcggat gcgaagaaca 6180
cggaagcctc tggaaacaaa ggcgacgcgg tctccatcct caaggcgctc tttgatcgcg 6240
taaagccggg tgactatgtc gggctgctcg cctatatcag gcgcgaccag cagacacggg 6300
actgggcaca gtccgttcgc atggagcttc gggatgctct gaaggtggca acagctgccg 6360
aattcggacc ccggttcctg cattcgacgg gccaggccta caagggcgga cccgacagtg 6420
gcgtgttcct gcaggttacg gcgacggatg cggcggatgt gccggttccg ggccatggct 6480
tcagcttcag tgtcgtgaaa gcggcacagg cgcgaggcga tttcgaggtg ctcgcggccc 6540

gtggtcgtcg tgccctccgc gttcatatcg atggaccgct ggaggacggg ctgaaggctc 6600
tggaaacggc gatccacaaa gcactggcag gagcataaaa atcgaatact tatggcagca 6660
gaaccctgac cgagtggctc acactggtcc taggggttgt catcattcac atgaagcacg 6720
gccagaaggt gctgtggcag cacccgctgg gaacggcccg cgccaatggt ccgtggggcc 6780
tgccgaccgg tcttccctgg gaaatcggta cgccgaataa tggtggctcg gtcgtgacgg 6840
ccggtggcgt ggtgttcatc gcggcagcta cggataacca gatccgtgcc atcgacgagc 6900
acaccggcaa ggtggtctgg agcgcggtcc tgccgggcgg cggtcaggct aacccgatga 6960
cctacgaagc caatggtcat cagtacgtcg ccatcatggc gggtggtcat cacttcatga 7020
tgacgccggt cagcgatcag ctggtggttt acgcactgcc tgatcacaag ggctgagaaa 7080
gcgggggctg aggccgccgg gtgatggaag ggtggtttt cgtgctgcaa atggcacgga 7140
gaccgcccct ttgtcgtttc cggggccgtt ttttccagaa ttcgatatca agcttatcga 7200
taccgtcgac ctcgaggggg ggcccggtac ccagcttttg ttccctttag tgagggttaa 7260
ttgcgcgctt ggcgtaatca tggtcatagc tgtttcctgt gtgaaattgt tatccgctca 7320
caattccaca caacatacga gccggaagca taaagtgtaa agcctggggt gcctaatgag 7380
tgagctaact cacattaatt gcgttgcgct cactgcccgc tttccagtcg ggaaacctgt 7440
cgtgccagct gcattaatga atcggccaac gcgcggggag aggcggtttg cgtattgggc 7500
gcatgcataa aaactgttgt aattcattaa gcattctgcc gacatggaag ccatcacaaa 7560
cggcatgatg aacctgaatc gccagcggca tcagcacctt gtcgccttgc gtataatatt 7620
tgcccatggg ggtgggcgaa gaactccagc atgagatccc cgcgctggag gatcatccag 7680
ccggcgtccc ggaaaacgat tccgaagccc aacctttcat agaaggcggc ggtggaatcg 7740
aaatctcgtg atggcaggtt gggcgtcgct tggtcggtca tttcgaaccc cagagtcccg 7800
ctcagaagaa ctcgtcaaga aggcgataga aggcgatgcg ctgcgaatcg ggagcggcga 7860
taccgtaaag cacgaggaag cggtcagccc attcgccgcc aagctcttca gcaatatcac 7920
gggtagccaa cgctatgtcc tgatagcggt ccgccacacc cagccggcca cagtcgatga 7980
atccagaaaa gcggccattt tccaccatga tattcggcaa gcaggcatcg ccatgggtca 8040
cgacgagatc ctcgccgtcg ggcatgcgcg ccttgagcct ggcgaacagt tcggctggcg 8100
cgagcccctg atgctcttcg tccagatcat cctgatcgac aagaccggct tccatccgag 8160
tacgtgctcg ctcgatgcga tgtttcgctt ggtggtcgaa tgggcaggta gccggatcaa 8220
gcgtatgcag ccgccgcatt gcatcagcca tgatggatac tttctcggca ggagcaaggt 8280
gagatgacag gagatcctgc cccggcactt cgcccaatag cagccagtcc cttcccgctt 8340

```
cagtgacaac gtcgagcaca gctgcgcaag gaacgcccgt cgtggccagc cacgatagcc   8400
gcgctgcctc gtcctgcagt tcattcaggg caccggacag gtcggtcttg acaaaaagaa   8460
ccgggcgccc ctgcgctgac agccggaaca cggcggcatc agagcagccg attgtctgtt   8520
gtgcccagtc atagccgaat agcctctcca cccaagcggc cggagaacct gcgtgcaatc   8580
catcttgttc aatcatgcga aacgatcctc atcctgtctc ttgatcagat cttgatcccc   8640
tgcgccatca gatccttggc ggcaagaaag ccatccagtt tactttgcag ggcttcccaa   8700
ccttaccaga gggcgcccca gctggcaatt ccggttcgct tgctgtccat aaaaccgccc   8760
agtctagcta tcgccatgta agcccactgc aagctacctg ctttctcttt gcgcttgcgt   8820
tttcccttgt ccagatagcc cagtagctga cattcatccc aggtggcact tttcggggaa   8880
atgtgcgcgc ccgcgttcct gctggcgctg ggcctgtttc tggcgctgga cttcccgctg   8940
ttccgtcagc agcttttcgc ccacggcctt gatgatcgcg gcggccttgg cctgcatatc   9000
ccgattcaac ggccccaggg cgtccagaac gggcttcagg cgctcccgaa ggt          9053
```

## Description

The present invention relates to microorganisms genetically engineered to increase yield and/or efficiency of biomass production from a carbon source such as e.g. sorbitol. Processes for generating such microorganisms are also provided by the present invention. The invention also relates to polynucleotide sequences comprising genes that encode proteins that are involved in the bioconversion of a carbon source such as e.g. sorbitol into biomass. The invention also features polynucleotides comprising the full-length polynucleotide sequences of the novel genes and fragments thereof, the novel polypeptides encoded by the polynucleotides and fragments thereof, as well as their functional equivalents. Also included are processes of using the polynucleotides and modified polynucleotide sequences to transform host microorganisms leading to a microorganism with higher yield and/or efficiency of biomass production from a carbon source such as e.g. sorbitol.

The bioconversion of a carbon source may involve many different metabolic routes, and involve several enzymatic steps to generate biomass, wherein the enzymes may be located in the cytosol, on the membrane or in the periplasmic space of a host cell. Furthermore, transporters may also play an important role in the efficient conversion of a carbon source into biomass.

For instance, in the case of acetic acid bacteria, which are obligate aerobe, gram-negative microorganisms belonging to the genus *Acetobacter, Gluconobacter,* and *Gluconacetobacter,* these microorganisms are able to rapidly perform incomplete oxidation of sugars and sugar alcohols at the periplasmic membrane level. The resulting products (e.g. organic acids, aldehydes or ketones) are predominantly secreted in the medium. In addition to that, sugars and sugar alcohols can be directly transported into the cytosol and converted into metabolites and biomass. Acetic acid bacteria lack the Embden-Meyerhof-Parnas pathway of glycolysis. Carbon sources can be converted to glucose-6-phosphate or 6-phosphogluconate and metabolized via the pentose phosphate pathway or the Entner-Doudoroff pathway to produce reducing power in the form of NAD(P)H, energy in form of ATP and tricarboxylic compounds necessary for growth and maintenance.

One disadvantage of such bioconversion processes, however, is the diversion of carbon sources or intermediates throughout said bioconversion process such that for instance the next (enzymatic) step cannot be performed in an optimal way, leading to the loss of available carbon substrate material for conversion into biomass and resultant energy losses, in form of e.g. ATP or NADPH. In the case of for instance the bioconversion of glucose into biomass, this loss may be due to transporting the glucose or an intermediate thereof out of the cytosol or by using the glucose or the resulting intermediates as substrates for other pathways not leading to the production of biomass.

It is an object of the present invention to provide microorganisms which are engineered in such a way that the carbon source flow throughout the bioconversion of carbon sources, such as e.g. sorbitol, is altered, e.g. via a reduction of carbon source diversion, leading to higher production and/or yield of biomass produced from such carbon sources.

Surprisingly, it has been found that proteins or subunits of such proteins having activity towards or which are involved in the metabolization of substrates such as e.g. sorbitol, which are selected from isomerases [EC 5], preferably intramolecular oxidoreductases [EC 5.3], more preferably intramolecular oxidoreductases interconverting aldoses and ketoses [EC 5.3.1], and most preferably selected from glucose-6-phosphate isomerases [EC 5.3.1.9] referred herein as to pgi, play an important role in the formation of biomass.

In particular, it has now been found that proteins encoded by polynucleotides having a nucleotide sequence that hybridizes preferably under highly stringent conditions to a sequence shown in SEQ ID NO: 1 play an important role in the bioconversion of a carbon source such as e.g. sorbitol to biomass. It has also been found, that by genetically altering such nucleotides in a microorganism, such as for example *Gluconobacter,* the efficiency of said bioconversion within said microorganism can be even greatly improved leading e.g. to higher production and/or yield of biomass from a carbon source such as e.g. sorbitol.

Consequently, the invention relates to a polynucleotide selected from the group consisting of:

(a) polynucleotides encoding a polypeptide comprising the amino acid sequence according to SEQ ID NO:2;

(b) polynucleotides comprising the nucleotide sequence according to SEQ ID NO: 1;

(c) polynucleotides comprising a nucleotide sequence obtainable by nucleic acid amplification such as polymerase chain reaction, using genomic DNA from a microorganism as a template and a primer set according to SEQ ID NO: 3 and SEQ ID NO:4;

(d) polynucleotides comprising a nucleotide sequence encoding a fragment or derivative of a polypeptide encoded by a polynucleotide of any of (a) to (c) wherein in said derivative one or more amino acid residues are conservatively substituted compared to said polypeptide, and said fragment or derivative has the activity of an isomerase [EC 5], preferably an intramolecular oxidoreductase [EC 5.3], most preferably pgi;

(e) polynucleotides the complementary strand of which hybridizes under stringent conditions to a polynucleotide as defined in any one of (a) to (d) and which encode an isomerase [EC 5], preferably an intramolecular oxidoreductase [EC 5.3], most preferably pgi;

(f) polynucleotides which are at least 70%, such as 85, 90 or 95% identical to a polynucleotide as defined in any one of (a) to (d) and which encode an isomerase [EC 5], preferably an intramolecular oxidoreductase [EC 5.3], most preferably pgi;

or
the complementary strand of such a polynucleotide.

The polypeptide as isolated from *Gluconobacter oxydans* DSM 2343 (ATCC 621H) shown in SEQ ID NO:2 and described herein was found to be particularly useful, since it appeared that it performs a crucial function in the bioconversion of a carbon source such as e.g. sorbitol to biomass in microorganisms, in particular in bacteria, such as acetic acid bacteria, such as *Gluconobacter, Acetobacter* and *Gluconacetobacter.* Accordingly, the invention relates to a polynucleotide encoding a polypeptide according to SEQ ID NO:2. This protein may be encoded by a nucleotide sequence as shown in SEQ ID NO:1. The invention therefore also relates to polynucleotides comprising the nucleotide sequence according to SEQ ID NO:1.

The nucleotide and amino acid sequences determined above were used as a "query sequence" to perform a search with Blast2 program (version 2 or BLAST from National Center for Biotechnology [NCBI] against the database PRO SW-SwissProt (full release plus incremental updates). From the searches, the polynucleotide according to SEQ ID NO: 1 was annotated as encoding a protein having glucose-6-phosphate isomerase (pgi) activity.

A nucleic acid according to the invention may be obtained by nucleic acid amplification using cDNA, mRNA or alternatively, genomic DNA, as a template and appropriate oligonucleotide primers such as the nucleotide primers according to SEQ ID NO:3 and SEQ ID NO:4 according to standard PCR amplification techniques. The nucleic acid thus amplified may be cloned into an appropriate vector and characterized by DNA sequence analysis.

The template for the reaction may be cDNA obtained by reverse transcription of mRNA prepared from strains known or suspected to comprise a polynucleotide according to the invention. The PCR product may be subcloned and sequenced to ensure that the amplified sequences represent the sequences of a new nucleic acid sequence as described herein, or a functional equivalent thereof.

The PCR fragment may then be used to isolate a full length cDNA clone by a variety of known methods. For example, the amplified fragment may be labeled and used to screen a bacteriophage or cosmid cDNA library. Alternatively, the labeled fragment may be used to screen a genomic library.

Accordingly, the invention relates to polynucleotides comprising a nucleotide sequence obtainable by nucleic acid amplification such as polymerase chain reaction, using DNA such as genomic DNA from a microorganism as a template and a primer set according to SEQ ID NO:3 and SEQ ID NO:4.

The invention also relates to polynucleotides comprising a nucleotide sequence encoding a fragment or derivative of a polypeptide encoded by a polynucleotide as described herein wherein in said derivative one or more amino acid residues are conservatively substituted compared to said polypeptide, and said fragment or derivative has the activity of an isomerase, preferably pgi.

The invention also relates to polynucleotides the complementary strand of which hybridizes under stringent conditions to a polynucleotide as defined herein and which encode an isomerase, preferably pgi.

The invention also relates to polynucleotides which are at least 70% identical to a polynucleotide as defined herein and which encode an isomerase; and the invention also relates to polynucleotides being the complementary strand of a polynucleotide as defined herein above.

Such a polynucleotide may be for instance isolated from *Gluconobacter oxydans* DSM 17078 as shown in SEQ ID NO:5 and encoding a polypeptide according to SEQ ID NO:6. The invention therefore also relates to polynucleotides selected from the group consisting of

(a) comprising the nucleotide sequence according to SEQ ID NO:5;

(b) polynucleotides encoding a polypeptide comprising the amino acid sequence according to SEQ ID NO:6;

(c) polynucleotides comprising a nucleotide sequence obtainable by nucleic acid amplification such as polymerase chain reaction, using genomic DNA from a microorganism as a template and a primer set according to SEQ ID NO: 7 and SEQ ID NO:8;

(d) polynucleotides comprising a nucleotide sequence encoding a fragment or derivative of a polypeptide encoded by a polynucleotide of any of (a) to (c) wherein in said derivative one or more amino acid residues are conservatively substituted compared to said polypeptide, and said fragment or derivative has the activity of an isomerase [EC 5], preferably an intramolecular oxidoreductase [EC 5.3], most preferably pgi;

(e) polynucleotides the complementary strand of which hybridizes under stringent conditions to a polynucleotide as defined in any one of (a) to (d) and which encode an isomerase [EC 5], preferably an intramolecular oxidoreductase [EC 5.3], most preferably pgi;

(f) polynucleotides which are at least 70%, such as 85, 90 or 95% identical to a polynucleotide as defined in any one of (a) to (d) and which encode an isomerase [EC 5], preferably an intramolecular oxidoreductase [EC 5.3], most preferably pgi;

or
the complementary strand of such a polynucleotide.

In one embodiment the polypeptides as of the present invention may be present in the form of a fusion protein, such as *e.g.* pgi fused to a further protein, such as *e.g.* a transferase [EC 2], in particular a transaldolase [EC 2.2.1.2], referred hereafter as to tal. A polynucleotide expressing such a fusion of pgi and tal as isolated from *Gluconobacter oxydans* DSM 2343 is shown in SEQ ID NO:9, the respective polypeptide is shown in SEQ ID NO:10.

The invention also relates to microorganisms wherein the activity of an isomerase, preferably pgi, is enhanced and/or improved so that the yield and/or production of biomass which is produced through bioconversion of a carbon source such as such as e.g. sorbitol is increased.

A suitable carbon source that can be converted into biomass may be for instance sorbitol, glucose, fructose, mannitol, sorbose, glycerol, or may be selected from carbon sources the assimilation of which results in the formation of said substances, such as sucrose, maltose or starch. Mixtures of the above carbon sources may be also used for the purpose of the present invention, such as glucose and sorbitol, or fructose and sorbitol. Preferably, the carbon source is selected from glucose, fructose, sorbitol, mannitol, sorbose, glycerol, sucrose, and mixtures thereof, wherein sorbitol or mixtures containing sorbitol are most preferred.

The skilled person will know how to enhance and/or improve the activity of an isomerase, preferably pgi. Such may be for instance accomplished by either genetically modifying the host organism in such a way that it produces more or more stable copies of such protein, preferably pgi, than the wild type organism or by increasing the specific activity of such protein, preferably the pgi.

In the following description, procedures are detailed to achieve this goal, *i.e.* the increase in the yield and/or production of biomass which is produced through bioconversion of a carbon source such as e.g. sorbitol by increasing the activity of pgi. These procedures apply mutatis mutandis for other isomerases or fusion proteins comprising pgi and tal.

Modifications in order to have the organism produce more copies of the pgi gene and/or protein may include the use of a strong promoter, or the mutation (e.g. insertion, deletion or point mutation) of (parts of) the pgi gene or its regulatory elements. It may also involve the insertion of multiple copies of the gene into a suitable microorganism. An increase in the specific activity of a pgi may also be accomplished by methods known in the art. Such methods may include the mutation (e.g. insertion, deletion or point mutation) of (parts of) the pgi gene. A gene is said to be "overexpressed" if the level of transcription of said gene is enhanced in comparison to the wild type gene. This may be measured by for instance Northern blot analysis quantifying the amount of mRNA as an indication for gene expression. As used herein, a gene is overexpressed if the amount of generated mRNA is increased by at least 1%, 2%, 5% 10%, 25%, 50%, 75%, 100%, 200% or even more than 500%, compared to the amount of mRNA generated from a wild-type gene.

Also known in the art are methods of increasing the activity of a given protein by contacting pgi with specific enhancers or other substances that specifically interact with pgi. In order to identify such specific enhancers, pgi may be expressed and tested for activity in the presence of compounds suspected to enhance the activity of pgi. The activity of pgi may also be increased by stabilizing the messenger RNA encoding pgi. Such methods are also known in the art, see for example, in Sambrook et al., 1989, Molecular Cloning, A Laboratory Manual, Cold Spring Harbor Press, N. Y.; and Ausubel et al. (eds.), 1995, Current Protocols in Molecular Biology, (John Wiley & Sons, N.Y.).

The invention may be performed in any microorganism carrying a pgi gene or homologue thereof. Suitable microorganisms may be selected from the group consisting of yeast, algae and bacteria, either as wild type strains, mutant strains derived by classic mutagenesis and selection methods or as recombinant strains. Examples of such yeast may be, *e.g., Candida, Saccharomyces, Zygosaccharomyces, Schizosaccharomyces,* or *Kluyveromyces.* An example of such algae may be, *e.g., Chlorella.* Examples of such bacteria may be, *e.g., Gluconobacter, Acetobacter, Gluconacetobacter, Ketogulonicigenium, Pantoea, Pseudomonas,* such as, *e.g., Pseudomonas putida,* and *Escherichia,* such as, *e.g., Escherichia coli.* Preferred are *Gluconobacter* or *Acetobacter aceti,* such as for instance *G. oxydans, G. cerinus, G. frateurii, A. aceti subsp. xylinum* or *A. aceti subsp. orleanus,* preferably *G. oxydans* DSM 17078 or G. *oxydans* DSM 2343 (ATCC 621H). *Gluconobacter oxydans* DSM 17078 (formerly known as *Gluconobacter oxydans* N44-1) has been deposited at Deutsche Sammlung von Mikroorganismen und Zellkulturen (DSMZ), Mascheroder Weg 1B, D-38124 Braunschweig, Germany according to the Budapest Treaty on 26. January 2005.

Microorganisms which can be used for the present invention may be publicly available from different sources, e.g., Deutsche Sammlung von Mikroorganismen und Zellkulturen (DSMZ), Mascheroder Weg 1B, D-38124 Braunschweig, Germany, American Type Culture Collection (ATCC), P.O. Box 1549, Manassas, VA 20108 USA or Culture Collection Division, NITE Biological Resource Center, 2-5-8, Kazusakamatari, Kisarazushi, Chiba, 292-0818, Japan (formerly: Institute for Fermentation, Osaka (IFO), 17-85, Juso-honmachi 2-chome,Yodogawa-ku, Osaka 532-8686, Japan). Examples of preferred bacteria deposited with IFO are for instance *Gluconobacter oxydans* (formerly known as *G. melanogenus)* IFO 3293, *Gluconobacter oxydans* (formerly known as *G. melanogenus)* IFO 3292, *Gluconobacter oxydans* (formerly known as *G. rubiginosus)* IFO 3244, *Gluconobacter frateurii* (formerly known as *G. industrius)* IFO 3260, *Gluconobacter cerinus* IFO 3266, *Gluconobacter oxydans* IFO 3287, and *Acetobacter aceti subsp. orleanus* IFO 3259, which were all deposited on April 05, 1954; *Acetobacter aceti subsp. xylinum* IFO 13693 deposited on October 22, 1975, and *Acetobacter aceti subsp. xylinum* IFO 13773 deposited on December 08, 1977. Strain *Acetobacter sp.* ATCC 15164, which is also an example of a preferred bacterium, was deposited with ATCC. Strain *Gluconobacter oxydans* (formerly known as *G. melanogenus)* N 44-1 as another example of a preferred bacterium is a derivative of the strain IFO 3293 and is described in Sugisawa et al., Agric. Biol. Chem. 54: 1201-1209, 1990.

A microorganism as of the present invention may carry further modifications either on the DNA or protein level (see above), as long as such modification(s) has/have a direct impact on the yield, production and/or efficiency of biomass from a substrate such as e.g. sorbitol. Such further modifications may for instance affect other genes encoding isomerases as described above, in particular genes encoding ribulose-5-phosphate isomerase, or further genes encoding enzymes involved in the pentose phosphate pathway, such as for instance glucose-6-phosphate dehydrogenase, 6-phosphogluconate dehydrogenase, 6-phosphogluconolactonase, ribulose-5-phosphate 3-epimerase, transaldolase or transketolase, or further genes encoding enzymes linking the pentose phosphate pathway to other pathways of carbon metabolism, such as for instance 6-phosphogluconate dehydratase, fructose -1,6-bisphosphate or phosphofructokinase. Methods of performing such modifications are known in the art, with some examples further described herein.

In accordance with a further object of the present invention there is provided the use of a polynucleotide as defined above or a microorganism which is genetically engineered using such polynucleotides in the efficient production of biomass from a carbon source.

The invention also relates to processes for the expression of endogenous genes in a microorganism, to processes for the production of polypeptides as defined above in a microorganism and to processes for the production of microorganisms capable of growth on a given carbon source such as e.g. sorbitol. All these processes comprise the step of altering a microorganism, wherein "altering" as used herein encompasses the process for "genetically altering" or "altering the composition of the cell culture media and/or methods used for culturing" in such a way that the yield and/or productivity of biomass can be improved compared to the wild-type organism. As used herein, "improved yield of biomass" means an increase of at least 5%, 10%, 25%, 30%, 40%, 50%, 75%, 100%, 200% or even more than 500%, depending on the carbon source used for growth, compared to a wild-type microorganism, *i.e.* a microorganism which is not genetically altered.

The term "genetically engineered" or "genetically altered" means the scientific alteration of the structure of genetic material in a living organism. It involves the production and use of recombinant DNA. More in particular it is used to delineate the genetically engineered or modified organism from the naturally occurring organism. Genetic engineering may be done by a number of techniques known in the art, such as e.g. gene replacement, gene amplification, gene disruption, transfection, transformation using plasmids, viruses, or other vectors. A genetically modified organism, e.g. genetically modified microorganism, is also often referred to as a recombinant organism, e.g. recombinant microorganism.

Advantageous embodiments of the invention become evident from the dependent claims. These and other aspects and embodiments of the present invention should now be apparent to those skilled in the art based on the teachings herein.

The sequence of the gene comprising a nucleotide sequence according to SEQ ID NO:1 encoding pgi was determined by sequencing a genomic clone obtained from *Gluconobacter oxydans* DSM 2343.

The invention also relates to a polynucleotide encoding at least a biologically active fragment or derivative of pgi as shown in SEQ ID NO:2.

As used herein, "biologically active fragment or derivative" means a polypeptide which retains essentially the same biological function or activity as the polypeptide shown in SEQ ID NO:2. Examples of biological activity may for instance be enzymatic activity, signaling activity or antibody reactivity. The term "same biological function" or "functional equivalent" as used herein means that the protein has essentially the same biological activity, e.g. enzymatic, signaling or antibody reactivity, as a polypeptide shown in SEQ ID NO:2.

The polypeptides and polynucleotides of the present invention are preferably provided in an isolated form, and preferably are purified to homogeneity.

The term "isolated" means that the material is removed from its original environment (e.g., the natural environment if it is naturally occurring). For example, a naturally-occurring polynucleotide or polypeptide present in a living microorganism is not isolated, but the same polynucleotide or polypeptide, separated from some or all of the coexisting materials in the natural system, is isolated. Such polynucleotides could be part of a vector and/or such polynucleotides or polypeptides could be part of a composition and still be isolated in that such vector or composition is not part of its natural environment.

An isolated polynucleotide or nucleic acid as used herein may be a DNA or RNA that is not immediately contiguous with both of the coding sequences with which it is immediately contiguous (one on the 5'-end and one on the 3'-end) in the naturally occurring genome of the organism from which it is derived. Thus, in one embodiment, a nucleic acid includes some or all of the 5'-non-coding *(e.g.,* promoter) sequences that are immediately contiguous to the coding sequence. The term "isolated polynucleotide" therefore includes, for example, a recombinant DNA that is incorporated into a vector, into an autonomously replicating plasmid or virus, or into the genomic DNA of a prokaryote or eukaryote, or which exists as a separate molecule (e.g., a cDNA or a genomic DNA fragment produced by PCR or restriction endonuclease treatment) independent of other sequences. It also includes a recombinant DNA that is part of a hybrid gene encoding an additional polypeptide that is substantially free of cellular material, viral material, or culture medium (when produced by recombinant DNA techniques), or chemical precursors or other chemicals (when chemically synthesized). Moreover, an "isolated nucleic acid fragment" is a nucleic acid fragment that is not naturally occurring as a fragment and would not be found in the natural state.

As used herein, the terms "polynucleotide", "gene" and "recombinant gene" refer to nucleic acid molecules which may be isolated from chromosomal DNA, which include an open reading frame encoding a protein, *e.g. G. oxydans* DSM 2343 isomerases. A polynucleotide may include a polynucleotide sequence as shown in SEQ ID NO: 1 or fragments thereof and regions upstream and downstream of the gene sequences which may include, for example, promoter regions, regulator regions and terminator regions important for the appropriate expression and stabilization of the polypeptide derived thereof.

A gene may include coding sequences, non-coding sequences such as for instance untranslated sequences located at the 3'- and 5'-ends of the coding region of a gene, and regulatory sequences. Moreover, a gene refers to an isolated nucleic acid molecule as defined herein. It is furthermore appreciated by the skilled person that DNA sequence polymorphisms that lead to changes in the amino acid sequences of isomerases may exist within a population, e.g., the *Gluconobacter oxydans* population. Such genetic polymorphism in the pgi gene may exist among individuals within a population due to natural variation or in cells from different populations. Such natural variations can typically result in 1-5% variance in the nucleotide sequence of the pgi gene. Any and all such nucleotide variations and the resulting amino acid polymorphism in pgi are the result of natural variation and that do not alter the function or biological activity of isomerases are intended to be within the scope of the invention.

As used herein, the terms "polynucleotide" or "nucleic acid molecule" are intended to include DNA molecules (e.g., cDNA or genomic DNA) and RNA molecules (e.g., mRNA) and analogs of the DNA or RNA generated using nucleotide analogs. The nucleic acid molecule may be single-stranded or double-stranded, but preferably is double-stranded DNA. The nucleic acid may be synthesized using oligonucleotide analogs or derivatives (e.g., inosine or phosphorothioate nucleotides). Such oligonucleotides may be used, for example, to prepare nucleic acids that have altered base-pairing abilities or increased resistance to nucleases.

The sequence information as provided herein should not be so narrowly construed as to require inclusion of erroneously identified bases. The specific sequences disclosed herein may be readily used to isolate the complete gene from a microorganism capable of converting a given carbon source such as e.g. sorbitol into biomass, in particular *Gluconobacter oxydans,* preferably *Gluconobacter oxydans* DSM 17078 or *Gluconobacter oxydans* DSM 2343 which in turn may easily be subjected to further sequence analyses thereby identifying sequencing errors.

Unless otherwise indicated, all nucleotide sequences determined by sequencing a DNA molecule herein were determined using an automated DNA sequencer and all amino acid sequences of polypeptides encoded by DNA molecules determined herein were predicted by translation of a DNA sequence determined as above. Therefore, as is known in the art for any DNA sequence determined by this automated approach, any nucleotide sequence determined herein may contain some errors. Nucleotide sequences determined by automation are typically at least about 90% identical, more typically at least about 95% to at least about 99.9% identical to the actual nucleotide sequence of the sequenced DNA molecule. The actual sequence may be more precisely determined by other approaches including manual DNA sequencing methods well known in the art. As is also known in the art, a single insertion or deletion in a determined nucleotide sequence compared to the actual sequence will cause a frame shift in translation of the nucleotide sequence such that the predicted amino acid sequence encoded by a determined nucleotide sequence will be completely different from the amino acid sequence actually encoded by the sequenced DNA molecule, beginning at the point of such an insertion or deletion.

The person skilled in the art is capable of identifying such erroneously identified bases and knows how to correct for such errors.

A nucleic acid molecule according to the invention may comprise only a portion or a fragment of the nucleic acid sequence provided by the present invention, such as for instance the sequence shown in SEQ ID NO:1, for example a fragment which may be used as a probe or primer such as for instance SEQ ID NO:3 or SEQ ID NO:4 or a fragment encoding a portion of a protein according to the invention. The nucleotide sequence determined from the cloning of the pgi gene allows for the generation of probes and primers designed for use in identifying and/or cloning other pgi family members, as well as pgi homologues from other species. The probe/primer typically comprises substantially purified oligonucleotides which typically comprises a region of nucleotide sequence that hybridizes preferably under highly stringent conditions to at least about 12 or 15, preferably about 18 or 20, more preferably about 22 or 25, even more preferably about 30, 35, 40, 45, 50, 55, 60, 65, or 75 or more consecutive nucleotides of a nucleotide sequence shown in SEQ ID NO: 1 or a fragment or derivative thereof.

A nucleic acid molecule encompassing all or a portion of the nucleic acid sequence of SEQ ID NO: 1 may be isolated by the polymerase chain reaction (PCR) using synthetic oligonucleotide primers designed based upon the sequence information contained herein.

A nucleic acid of the invention may be amplified using cDNA, mRNA or alternatively, genomic DNA, as a template and appropriate oligonucleotide primers according to standard PCR amplification techniques. The nucleic acid thus amplified may be cloned into an appropriate vector and characterized by DNA sequence analysis.

Fragments of a polynucleotide according to the invention may also comprise polynucleotides not encoding functional polypeptides. Such polynucleotides may function as probes or primers for a PCR reaction.

Nucleic acids according to the invention irrespective of whether they encode functional or non-functional polypeptides, may be used as hybridization probes or polymerase chain reaction (PCR) primers. Uses of the nucleic acid molecules of the present invention that do not encode a polypeptide having a pgi activity include, inter alia, (1) isolating the gene encoding the protein of the present invention, or allelic variants thereof from a cDNA library, e.g., from other organisms than *Gluconobacter oxydans* and (2) Northern blot analysis for detecting expression of mRNA of said protein in specific cells or (3) use in abolishing or altering the function or activity of homologous pgi genes in said other organisms.

Probes based on the nucleotide sequences provided herein may be used to detect transcripts or genomic sequences encoding the same or homologous proteins for instance in other organisms. Nucleic acid molecules corresponding to natural variants and non-G. *oxydans* homologues of the *G. oxydans* pgi DNA of the invention which are also embraced by the present invention may be isolated based on their homology to the *G. oxydans* pgi nucleic acid disclosed herein using the *G. oxydans* DNA, or a portion thereof, as a hybridization probe according to standard hybridization techniques, preferably under highly stringent hybridization conditions.

In preferred embodiments, the probe further comprises a label group attached thereto, e.g., the label group can be a radioisotope, a fluorescent compound, an enzyme, or an enzyme cofactor.

Homologous gene sequences may be isolated, for example, by performing PCR using two degenerate oligonucleotide primer pools designed on the basis of nucleotide sequences as taught herein.

The template for the reaction may be cDNA obtained by reverse transcription of mRNA prepared from strains known or suspected to express a polynucleotide according to the invention. The PCR product may be subcloned and sequenced to ensure that the amplified sequences represent the sequences of a new nucleic acid sequence as described herein, or a functional equivalent thereof.

The PCR fragment may then be used to isolate a full length cDNA clone by a variety of known methods. For example, the amplified fragment may be labeled and used to screen a bacteriophage or cosmid cDNA library. Alternatively, the labeled fragment may be used to screen a genomic library.

PCR technology can also be used to isolate full-length cDNA sequences from other organisms. For example, RNA may be isolated, following standard procedures, from an appropriate cellular or tissue source. A reverse transcription reaction may be performed on the RNA using an oligonucleotide primer specific for the most 5'-end of the amplified fragment for the priming of first strand synthesis.

The resulting RNA/DNA hybrid may then be "tailed" (e.g., with guanines) using a standard terminal transferase reaction, the hybrid may be digested with RNaseH, and second strand synthesis may then be primed (e.g., with a poly-C primer). Thus, cDNA sequences upstream of the amplified fragment may easily be isolated. For a review of useful cloning strategies, see e.g., Sambrook et al., supra; and Ausubel et al., supra.

Also, nucleic acids encoding other pgi family members, which thus have a nucleotide sequence that differs from a nucleotide sequence according to SEQ ID NO:1, are within the scope of the invention. Moreover, nucleic acids encoding pgi proteins from different species which thus may have a nucleotide sequence which differs from a nucleotide sequence shown in SEQ ID NO:1 are within the scope of the invention.

The invention also relates to an isolated polynucleotide hybridisable under stringent conditions, preferably under highly stringent conditions, to a polynucleotide according to the present invention, such as for instance a polynucleotide shown in SEQ ID NO:1. Advantageously, such polynucleotide may be obtained from a microorganism capable of converting a given carbon source such as e.g. sorbitol into biomass, in particular *Gluconobacter oxydans,* preferably *Gluconobacter oxydans* DSM 17078 or *Gluconobacter oxydans* DSM 2343.

As used herein, the term "hybridizing" is intended to describe conditions for hybridization and washing under which nucleotide sequences at least about 50%, at least about 60%, at least about 70%, more preferably at least about 80%, even more preferably at least about 85% to 90%, most preferably at least 95% homologous to each other typically remain hybridized to each other.

In one embodiment, a nucleic acid of the invention is at least 40%, 45%, 50%, 55%, 60%, 65%, 70%, 75%, 80%, 85%, 90%, 91%, 92%, 93%, 94%, 95%, 96%, 97%, 98%, 99%, or more homologous to a nucleic acid sequence shown in SEQ ID NO: 1 or the complement thereof.

A preferred, non-limiting example of such hybridization conditions are hybridization in 6x sodium chloride/ sodium citrate (SSC) at about 45°C, followed by one or more washes in 1x SSC, 0.1% SDS at 50°C, preferably at 55°C, more preferably at 60°C and even more preferably at 65°C.

Highly stringent conditions include incubations at 42°C for a period of several days, such as 2-4 days, using a labeled DNA probe, such as a digoxigenin (DIG)-labeled DNA probe, followed by one or more washes in 2x SSC, 0.1% SDS at room temperature and one or more washes in 0.5x SSC, 0.1% SDS or 0.1x SSC, 0.1% SDS at 65-68°C. In particular, highly stringent conditions include, for example, 2 h to 4 days incubation at 42°C using a DIG-labeled DNA probe (prepared by e.g. using a DIG labeling system; Roche Diagnostics GmbH, 68298 Mannheim, Germany) in a solution such as DigEasyHyb solution (Roche Diagnostics GmbH) with or without 100 μg/ml salmon sperm DNA, or a solution comprising 50% formamide, 5x SSC (150 mM NaCl, 15 mM trisodium citrate), 0.02% sodium dodecyl sulfate, 0.1 % N-lauroylsarcosine, and 2% blocking reagent (Roche Diagnostics GmbH), followed by washing the filters twice for 5 to 15 minutes in 2x SSC and 0.1% SDS at room temperature and then washing twice for 15-30 minutes in 0.5x SSC and 0.1% SDS or 0.1x SSC and 0.1% SDS at 65-68°C.

Preferably, an isolated nucleic acid molecule of the invention that hybridizes under preferably highly stringent conditions to a nucleotide sequence of the invention corresponds to a naturally-occurring nucleic acid molecule. As used herein, a "naturally-occurring" nucleic acid molecule refers to an RNA or DNA molecule having a nucleotide sequence that occurs in nature (e.g., encodes a natural protein). In one embodiment, the nucleic acid encodes a natural G. *oxydans* pgi.

The skilled artisan will know which conditions to apply for stringent and highly stringent hybridization conditions. Additional guidance regarding such conditions is readily available in the art, for example, in Sambrook et al., 1989, Molecular Cloning, A Laboratory Manual, Cold Spring Harbor Press, N. Y.; and Ausubel et al. (eds.), 1995, Current Protocols in Molecular Biology, (John Wiley & Sons, N. Y.). Of course, a polynucleotide which hybridizes only to a poly (A) sequence (such as the 3'-terminal poly (A) tract of mRNAs), or to a complementary stretch of T (or U) residues, would not be included in a polynucleotide of the invention used to specifically hybridize to a portion of a nucleic acid of the invention, since such a polynucleotide would hybridize to any nucleic acid molecule containing a poly (A) stretch or the complement thereof (e.g., practically any double-stranded cDNA clone).

In a typical approach, genomic DNA or cDNA libraries constructed from other organisms, e.g. microorganisms capable of converting of a given carbon source such as e.g. sorbitol into biomass, in particular other *Gluconobacter* species may be screened.

For example, *Gluconobacter* strains may be screened for homologous polynucleotides by Northern blot analysis. Upon detection of transcripts homologous to polynucleotides according to the invention, DNA libraries may be constructed from RNA isolated from the appropriate strain, utilizing standard techniques well known to those of skill in the art. Alternatively, a total genomic DNA library may be screened using a probe hybridisable to a polynucleotide according to the invention.

A nucleic acid molecule of the present invention, such as for instance a nucleic acid molecule shown in SEQ ID NO:1 or a fragment or derivative thereof, may be isolated using standard molecular biology techniques and the sequence information provided herein. For example, using all or portion of the nucleic acid sequence shown in SEQ ID NO: 1 as a hybridization probe, nucleic acid molecules according to the invention may be isolated using standard hybridization and cloning techniques (e.g., as described in Sambrook et al., Molecular Cloning : A Laboratory Manual. 2nd, ed. , Cold Spring Harbor Laboratory, Cold Spring Harbor Laboratory Press, Cold Spring Harbor, N.Y., 1989).

Furthermore, oligonucleotides corresponding to or hybridisable to nucleotide sequences according to the invention may be prepared by standard synthetic techniques, e.g., using an automated DNA synthesizer.

The terms "homology" or "percent identity" are used interchangeably herein. For the purpose of this invention, it is defined here that in order to determine the percent identity of two amino acid sequences or of two nucleic acid sequences, the sequences are aligned for optimal comparison purposes (e.g., gaps may be introduced in the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences *(i.e.,* % identity = number of identical positions/total number of positions *(i.e.,* overlapping positions) x 100). Preferably, the two sequences are the same length.

The skilled person will be aware of the fact that several different computer programs are available to determine the homology between two sequences. For instance, a comparison of sequences and determination of percent identity between two sequences may be accomplished using a mathematical algorithm. In a preferred embodiment, the percent identity between two amino acid sequences is determined using the Needleman and Wunsch (J. Mol. Biol. (48): 444-453 (1970)) algorithm which has been incorporated into the GAP program in the GCG software package (available at http: //www.accelrys.com), using either a Blossom 62 matrix or a PAM250 matrix, and a gap weight of 16, 14, 12, 10, 8, 6 or 4 and a length weight of 1, 2, 3, 4, 5 or 6. The skilled person will appreciate that all these different parameters will yield slightly different results but that the overall percentage identity of two sequences is not significantly altered when using different algorithms.

In yet another embodiment, the percent identity between two nucleotide sequences is determined using the GAP program in the GCG software package (available at http://www.accelrys.com), using a NWSgapdna.CMP matrix and a gap weight of 40, 50, 60, 70 or 80 and a length weight of 1, 2, 3, 4, 5 or 6. In another embodiment, the percent identity between two amino acid or nucleotide sequences is determined using the algorithm of E. Meyers and W. Miller (CABIOS, 4: 11-17 (1989) which has been incorporated into the ALIGN program (version 2.0) (available at http://vega.igh.cnrs.fr/bin/align-guess.cgi) using a PAM120 weight residue table, a gap length penalty of 12 and a gap penalty of 4.

The nucleic acid and protein sequences of the present invention may further be used as a "query sequence" to perform a search against public databases to, for example, identify other family members or related sequences. Such searches may be performed using the BLASTN and BLASTX programs (version 2.0) of Altschul, et al. (1990) J. Mol. Biol. 215:403-10. BLAST nucleotide searches may be performed with the BLASTN program, score = 100, word length = 12 to obtain nucleotide sequences homologous to the nucleic acid molecules of the present invention. BLAST protein searches may be performed with the BLASTX program, score = 50, word length = 3 to obtain amino acid sequences homologous to the protein molecules of the present invention. To obtain gapped alignments for comparison purposes, Gapped BLAST may be utilized as described in Altschul et al., (1997) Nucleic Acids Res. 25 (17): 3389-3402. When utilizing BLAST and Gapped BLAST programs, the default parameters of the respective programs (e.g., BLASTX and BLASTN) may be used. See http://www.ncbi.nlm.nih.gov.

In another preferred embodiment, an isolated nucleic acid molecule of the invention comprises a nucleic acid molecule which is the complement of a nucleotide sequence as of the present invention, such as for instance the sequence shown in SEQ ID NO: 1. A nucleic acid molecule, which is complementary to a nucleotide sequence disclosed herein, is one that is sufficiently complementary to a nucleotide sequence shown in SEQ ID NO: 1 such that it may hybridize to said nucleotide sequence thereby forming a stable duplex.

In a further preferred embodiment, a nucleic acid of the invention as shown in SEQ ID NO:1 or the complement thereof contains at least one mutation leading to a gene product with modified function/activity. The at least one mutation may be introduced by methods described herein. In one aspect, the at least one mutation leads to pgi whose function and/or activity compared to the wild type counterpart is enhanced or improved. Methods for introducing such mutations are well known in the art.

The term "increase" of activity as used herein encompasses increasing activity of one or more polypeptides in the producing organism, which in turn are encoded by the corresponding polynucleotides described herein. There are a number of methods available in the art to accomplish increase of activity of a given protein, in this case pgi. In general, the specific activity of a protein may be increased or the copy number of the protein may be increased. The term increase of activity or equivalent expressions also encompasses the situation wherein pgi activity is introduced in a cell that did not contain this activity before, e.g. by introducing a gene encoding pgi in a cell that did not contain an equivalent of this gene before, or that could not express an active form of the corresponding protein before.

To facilitate such an increase, the copy number of the genes corresponding to the polynucleotides described herein may be increased. Alternatively, a strong promoter may be used to direct the expression of the polynucleotide. In another embodiment, the promoter, regulatory region and/or the ribosome binding site upstream of the gene can be altered to increase the expression. The expression may also be enhanced or increased by increasing the relative half-life of the messenger RNA. In another embodiment, the activity of the polypeptide itself may be increased by employing one or more mutations in the polypeptide amino acid sequence, which increases the activity. For example, altering the affinity of the polypeptide for its corresponding substrate may result in improved activity. Likewise, the relative half-life of the polypeptide may be increased. In either scenario, that being enhanced gene expression or increased activity, the improvement may be achieved by altering the composition of the cell culture media and/or methods used for culturing. "Enhanced expression" or "improved activity" as used herein means an increase of at least 5%, 10%, 25%, 50%, 75%, or even 100%, compared to a wild-type protein, polynucleotide, gene; or the activity and/or the concentration of the protein present before the polynucleotides or polypeptides are enhanced and/or improved. The activity of pgi may also be enhanced by contacting the protein with a specific or general enhancer of its activity.

Another aspect of the invention pertains to vectors, containing a nucleic acid encoding a protein according to the invention or a functional equivalent or portion thereof. As used herein, the term "vector" refers to a nucleic acid molecule capable of transporting another nucleic acid to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (e.g., bacterial vectors having a bacterial origin of replication). Other vectors are integrated into the genome of a host cell upon introduction into the host cell, and thereby are replicated along with the host genome.

Moreover, certain vectors are capable of directing the expression of genes to which they are operatively linked. Such vectors are referred to herein as "expression vectors". In general, expression vectors of utility in recombinant DNA techniques are often in the form of plasmids. The terms "plasmid" and "vector" can be used interchangeably herein as the plasmid is the most commonly used form of vector. However, the invention is intended to include such other forms of expression vectors, such as viral vectors (e.g., replication defective retroviruses, adenoviruses and adeno-associated viruses), which serve equivalent functions.

The recombinant vectors of the invention comprise a nucleic acid of the invention in a form suitable for expression of the nucleic acid in a host cell, which means that the recombinant expression vector includes one or more regulatory sequences, selected on the basis of the host cells to be used for expression, which is operatively linked to the nucleic acid sequence to be expressed. Within a recombinant expression vector, "operatively linked" is intended to mean that the nucleotide sequence of interest is linked to the regulatory sequence(s) in a manner which allows for expression of the nucleotide sequence *(e.g.,* in an *in vitro* transcription/translation system or in a host cell when the vector is introduced into the host cell). The term "regulatory sequence" is intended to include promoters, enhancers and other expression control elements (e.g., attenuator). Such regulatory sequences are described, for example, in Goeddel; Gene Expression Technology: Methods in Enzymology 185, Academic Press, San Diego, CA (1990). Regulatory sequences include those which direct constitutive or inducible expression of a nucleotide sequence in many types of host cells and those which direct expression of the nucleotide sequence only in a certain host cell (e.g. tissue-specific regulatory sequences). It will be appreciated by those skilled in the art that the design of the expression vector can depend on such factors as the choice of the host cell to be transformed, the level of expression of protein desired, etc. The expression vectors of the invention may be introduced into host cells to thereby produce proteins or peptides, encoded by nucleic acids as described herein, including, but not limited to, mutant proteins, fragments thereof, variants or functional equivalents thereof, and fusion proteins, encoded by a nucleic acid as described herein, e.g., pgi proteins, mutant forms of pgi proteins, fusion proteins and the like.

The recombinant expression vectors of the invention may be designed for expression of pgi proteins in a suitable microorganism. For example, a protein according to the invention may be expressed in bacterial cells such as strains belonging to the genera *Gluconobacter, Gluconacetobacter or Acetobacter,* such as *e.g.* in G. *oxydans* DSM 17078 or G. *oxydans* DSM 2343. Expression vectors useful in the present invention include chromosomal-, episomal- and virus-derived vectors e.g., vectors derived from bacterial plasmids, bacteriophage, and vectors derived from combinations thereof, such as those derived from plasmid and bacteriophage genetic elements, such as cosmids and phagemids.

The DNA insert may be operatively linked to an appropriate promoter, which may be either a constitutive or inducible promoter. The skilled person will know how to select suitable promoters. The expression constructs may contain sites for transcription initiation, termination, and, in the transcribed region, a ribosome binding site for translation. The coding portion of the mature transcripts expressed by the constructs may preferably include an initiation codon at the beginning and a termination codon appropriately positioned at the end of the polypeptide to be translated.

Vector DNA may be introduced into suitable host cells via conventional transformation or transfection techniques. As used herein, the terms "transformation", "transconjugation" and "transfection" are intended to refer to a variety of art-recognized techniques for introducing foreign nucleic acid (e.g., DNA) into a host cell, including calcium phosphate or calcium chloride co-precipitation, DEAE-dextran-mediated transfection, transduction, infection, lipofection, cationic lipidmediated transfection or electroporation. Suitable methods for transforming or transfecting host cells may be found in Sambrook, et al. (supra), Davis et al., Basic Methods in Molecular Biology (1986) and other laboratory manuals.

In order to identify and select cells which have integrated the foreign DNA into their genome, a gene that encodes a selectable marker (e.g., resistance to antibiotics) is generally introduced into the host cells along with the gene of interest. Preferred selectable markers include those that confer resistance to drugs, such as kanamycin, tetracycline, ampicillin and streptomycin. A nucleic acid encoding a selectable marker is preferably introduced into a host cell on the same vector as that encoding a protein according to the invention or can be introduced on a separate vector such as, for example, a suicide vector, which cannot replicate in the host cells. Cells stably transfected with the introduced nucleic acid can be identified by drug selection (e.g., cells that have incorporated the selectable marker gene will survive, while the other cells die).

The invention provides also an isolated polypeptide having the amino acid sequence shown in SEQ ID NO:2 or an amino acid sequence obtainable by expressing a polynucleotide of the present invention, such as for instance a polynucleotide sequence shown in SEQ ID NO:1 in an appropriate host.

Polypeptides according to the invention may contain only conservative substitutions of one or more amino acids in the amino acid sequence represented by SEQ ID NO:2 or substitutions, insertions or deletions of non-essential amino acids. Accordingly, a non-essential amino acid is a residue that may be altered in the amino acid sequences shown in SEQ ID NO:2 without substantially altering the biological function. For example, amino acid residues that are conserved among the proteins of the present invention, are predicted to be particularly unamenable to alteration. Furthermore, amino acids conserved among the proteins according to the present invention and other pgi proteins are not likely to be amenable to alteration.

The term "conservative substitution" is intended to mean that a substitution in which the amino acid residue is replaced with an amino acid residue having a similar side chain. These families are known in the art and include amino acids with basic side chains (e.g., lysine, arginine and histidine), acidic side chains (e.g., aspartic acid, glutamic acid), uncharged polar side chains (e.g., glycine, asparagine, glutamine, serine, threonine, tyrosine, cysteine), non-polar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

As mentioned above, the polynucleotides of the invention may be utilized in the genetic engineering of a suitable host cell to make it better and more efficient in the bioconversion of a carbon source such as *e.g.* sorbitol to biomass, *i.e.* reduction of carbon source diversion throughout the bioconversion process.

According to the invention a genetically engineered/recombinant host cell (also referred to as recombinant cell or transformed cell) may be produced carrying such a modified polynucleotide wherein the function of the linked protein is significantly modified in comparison to a wild-type cell such that the yield and/or productivity of biomass from a carbon source such as e.g. sorbitol is improved. The host cell may be selected from a microorganism capable of converting a given carbon source such as e.g. sorbitol into biomass, in particular *Gluconobacter oxydans,* preferably G. *oxydans* DSM 17078 or G. *oxydans* DSM 2343.

A "transformed cell" or "recombinant cell" is a cell into which (or into an ancestor of which) has been introduced, by means of recombinant DNA techniques, a nucleic acid according to the invention, or wherein the activity of pgi has been increased and/or enhanced. Suitable host cells include cells of microorganisms capable of converting a given carbon source such as e.g. sorbitol into biomass. In particular, these include strains from the genera *Pseudomonas, Pantoea, Escherichia, Corynebacterium, Ketogulonicigenium* and acetic acid bacteria like *e.g., Gluconobacter, Acetobacter* or *Gluconacetobacter,* preferably *Acetobacter* sp., *Acetobacter aceti, Gluconobacter frateurii, Gluconobacter cerinus, Gluconobacter thailandicus, Gluconobacter oxydans,* more preferably G. *oxydans,* most preferably selected from G. *oxydans* DSM 17078, G. *oxydans* DSM 2343 or G. *oxydans* IFO 3293.

Improved gene expression may also be achieved by modifying the pgi gene, e.g., by introducing one or more mutations into the pgi gene wherein said modification leads to pgi with a function which is significantly improved in comparison to the wild-type protein.

Therefore, in one other embodiment, the polynucleotide carrying the at least one mutation is derived from a polynucleotide as represented by SEQ ID NO: 1 or equivalents thereof.

A mutation as used herein may be any mutation leading to a more functional or more stable polypeptide, e.g. more functional or more stable pgi gene products. This may include for instance an alteration in the genome of a microorganism, which improves the synthesis of pgi or leads to the expression of pgi with an altered amino acid sequence whose function compared with the wild type counterpart having a non-altered amino acid sequence is improved and/or enhanced. The improvement may occur at the transcriptional, translational or post-translational level.

The alteration in the genome of the microorganism may be obtained e.g. by replacing through a single or double crossover recombination a wild type DNA sequence by a DNA sequence containing the alteration. For convenient selection of transformants of the microorganism with the alteration in its genome the alteration may, e.g. be a DNA sequence encoding an antibiotic resistance marker or a gene complementing a possible auxotrophy of the microorganism. Mutations include, but are not limited to, deletion-insertion mutations.

An alteration in the genome of the microorganism leading to a more functional polypeptide may also be obtained by randomly mutagenizing the genome of the microorganism using e.g. chemical mutagens, radiation or transposons and selecting or screening for mutants which are better or more efficient producers of biomass. Standard methods for screening and selection are known to the skilled person.

In a specific embodiment, it is desired to knockout or suppress a repressor of the pgi gene of the present invention, *i.e.*, wherein its repressor gene expression is artificially suppressed in order to improve the yield, productivity, and/or efficiency of production of the fermentation product when introduced into a suitable host cell. Methods of providing knockouts as well as microorganisms carrying such suppressed genes are well known in the art. The suppression of the repressor gene may be induced by deleting at least a part of the repressor gene or the regulatory region thereof. As used herein, "suppression of the gene expression" includes complete and partial suppression, as well as suppression under specific conditions and also suppression of the expression of either one of the two alleles.

The aforementioned mutagenesis strategies for pgi proteins may result in increased yield and/or production of biomass. This list is not meant to be limiting; variations on these mutagenesis strategies will be readily apparent to one of ordinary skill in the art. By these mechanisms, the nucleic acid and protein molecules of the invention may be utilized to generate microorganisms such as *Gluconobacter oxydans* or related strains of bacteria expressing mutated pgi nucleic acid and protein molecules such that the yield, efficiency and/or productivity of biomass production from a carbon source such as e.g. sorbitol is improved.

Biomass concentration can be measured using several methods known to the person skilled in the art, such as e.g. measuring the optical density of the respective cell suspensions at for instance a wavelength of 600 nm ($OD_{600}$), or measuring either the wet- or dry-cell mass concentration or by counting the numbers of cells of the respective cell suspension. Such methods of measurement are known in the art. Thus, the cell dry weight (CDW) [g/l] may be for instance measured on a dried and tared nitrocellulose filter with a pore size of e.g. 0.45 μm on which the culture broth is filtrated. After washing the filter with water, the weight of the dried filter is again determined and the CDW calculated as follows:

$$CDW = m(\text{dried filter after broth filtration}) - m(\text{dried filter before broth filtration})$$

In one aspect of the invention, microorganisms (in particular from the genera of *Gluconobacter, Gluconacetobacter* and *Acetobacter)* are provided that are able to perform such improved bioconversion. When measured by a method as described herein these organisms were found to have an improved capability to produce biomass from a carbon source such as e.g. sorbitol. Such may be achieved by increasing the activity of an isomerase, preferably pgi. The yield of biomass produced from a carbon source, such as for instance sorbitol, when measured according to this method after an incubation period of 60-72 hours may be about 0.1 g or more dry biomass/g carbon source, preferably about 0.2 g or more, or even about 0.3 g or more dry biomass/g carbon source, such as for instance about 0.4 g, 0.5 g, 0.6 g, 0.7 g, 0.8 g or 1.0 g or more dry biomass/g carbon source.

The recombinant microorganism carrying e.g. a modified pgi gene and which is able to produce biomass from a carbon source such as e.g. sorbitol in significantly higher yield, productivity, and/or efficiency may be cultured in an aqueous medium supplemented with appropriate nutrients under aerobic conditions. The cultivation may be conducted in batch, fed-batch, semi-continuous or continuous mode. The cultivation period may vary depending on for instance the host, pH, temperature and nutrient medium to be used, and is preferably about 1 to about 10 days when run in batch or fed-batch mode. The cultivation may be conducted at for instance a pH of about 4.0 to about 9.0, preferably about 5.0 to about 8.0. The preferred temperature range for carrying out the cultivation is from about 13°C to about 36°C, preferably from about 18°C to about 33°C. Usually, the culture medium may contain besides e.g. sorbitol as main carbon source other assimilable carbon sources, e.g., glucose, glycerol, mannitol, sorbose, erythritol, ribitol, xylitol, arabitol, inositol, dulcitol, ribose, fructose, sucrose and ethanol, preferably glucose, mannitol, fructose, glycerol and ethanol; and digestible nitrogen sources such as organic substances, e.g., peptone, yeast extract, baker's yeast, urea, amino acids, and corn steep liquor. Various inorganic substances may also be used as nitrogen sources, e.g., nitrates and ammonium salts. Furthermore, the culture/incubation medium usually may contain inorganic salts, e.g., magnesium sulfate, manganese sulfate, potassium phosphate, and calcium carbonate.

The nucleic acid molecules, polypeptides, vectors, primers, and recombinant microorganisms described herein may be used in one or more of the following methods: identification of *Gluconobacter oxydans* and related organisms; mapping of genomes of organisms related to *Gluconobacter oxydans;* identification and localization of *Gluconobacter oxydans* sequences of interest; evolutionary studies; determination of pgi protein regions required for function; modulation of pgi activity or function; modulation of the activity of isomerase pathway; and modulation of cellular production of biomass from a carbon source such as e.g. sorbitol.

The invention provides methods for screening molecules which modulate the activity of pgi, either by interacting with the protein itself or a substrate or binding partner of pgi, or by modulating the transcription or translation of a pgi nucleic acid molecule of the invention. In such methods, a microorganism expressing one or more pgi proteins of the invention is contacted with one or more test compounds, and the effect of each test compound on the activity or level of expression of pgi is assessed.

The biological, enzymatic or other activity of isomerases can be measured by methods well known to a skilled person, such as, for example, by incubating a cell fraction containing pgi in the presence of its substrate, fructose-6-phosphate, whereby the substrate-dependent formation of NADPH may be measured spectrophotometrically at 340 nm according to the method described by e.g. Sugiyama et al., Biosci. Biotechnol. Biochem., 67 (12), 2524-2532, 2003.

Thus, the present invention is directed to the use of a polynucleotide, polypeptide, vector, primer and recombinant microorganism as described herein in the production of biomass, *i.e.,* the bioconversion of a carbon source such as *e.g.* sorbitol into biomass. In a preferred embodiment, a modified polynucleotide, polypeptide, vector and recombinant microorganism as described herein is used for improving the yield, productivity, and/or efficiency of said biomass production.

The terms "production" or "productivity" are art-recognized and include the amount of biomass formed within a given time and a given cultivation volume (e.g., kg product per hour per liter) from a given amount or concentration of a carbon source such as e.g. sorbitol. The term "efficiency of production" includes the time required for a particular level of production to be achieved (for example, how long it takes for the cell to attain a particular rate of output of a product). The term "yield" is art-recognized and includes the efficiency of the conversion of the carbon source into biomass. This is generally written as, for example, kg biomass per kg carbon source. By "increasing the yield and/or production of biomass" it is meant that in a given amount of culture over a given amount of time the biomass is increased. By measuring the increase in biomass- and decrease in carbon source concentration in a growing culture, one can calculate the yield of biomass on consumed carbon source; *i.e.* the weight of biomass obtained by bioconverting a determined weight of carbon source such as e.g. sorbitol, defined as kg biomass per kg carbon source consumed. To determine the consumption of carbon source, the residual amount of carbon source in the growth medium can be measured by methods well known in the art (e.g. HPLC). The increase of biomass can be measured by methods well known in the art (e.g. optical density at 600 nm, or measurement of dry weight of a sample).

The terms "biosynthesis" or a "biosynthetic pathway" are art-recognized and include the synthesis of a compound/product, preferably an organic compound, by a cell from intermediate compounds in what may be a multistep and highly regulated process. The term "bioconversion" is art-recognized and includes the conversion of a carbon source such as e.g. sorbitol into a product, *i.e.* biomass, by means of one or more biosynthetic step(s) which involve one or more enzyme(s) and/or transporter(s). The language "metabolism" is art-recognized and includes the totality of the biochemical reactions that take place in an organism. The metabolism of a particular compound, then, (e.g., the metabolism of an amino acid such as glycine) comprises the overall biosynthetic, modification, and degradation pathways in the cell related to this compound. The language "transport" or "import" is art-recognized and includes the facilitated movement of one or more molecules across a cellular membrane through which the molecule would otherwise either be unable to pass or be passed inefficiently.

In one preferred embodiment, the present invention is related to a process for the production of biomass from a carbon source such as e.g. sorbitol wherein a modified polynucleotide sequence as described above is introduced into a suitable microorganism and the recombinant microorganism is cultured under conditions that allow the production of biomass in high productivity, yield, and/or efficiency.

Recombinant microorganisms according to the present invention carrying e.g. a modified isomerase gene, in particular a modified pgi gene, and which are able to produce biomass from a carbon source such as e.g. sorbitol in significantly higher yield, productivity, and/or efficiency may advantageously be used in a number of applications. One particularly suited application is a method for the production of Vitamin C and/or intermediates thereof such like 2-keto-L-gulonic acid (2-KGA).

Thus, it is an aspect of the present invention to provide a process for the production of Vitamin C and/or 2-KGA wherein a nucleotide according to the invention or a modified polynucleotide sequence as described above is introduced into a suitable microorganism as described above wherein a suitable carbon source such as e.g. sorbitol is efficiently converted into biomass, the recombinant microorganism is cultured under conditions that allow the production of Vitamin C and/or 2-KGA in high productivity, yield, and/or efficiency, the produced fermentation product is isolated from the culture medium and optionally further purified.

The carbon sources or mixtures thereof used for the production of (1) biomass and (2) Vitamin C and/or 2-KGA may be the same or may differ. In one preferred embodiment, the carbon source(s) used for the production of biomass is different from the carbon source(s) used for the direct production of Vitamin C and/or 2-KGA. These two different carbon sources may be used simultaneously or sequentially, wherein a first carbon source would be used for the production of biomass, and this biomass would then be used to convert a second carbon source into Vitamin C and/or 2-KGA.

A suitable carbon source that can be converted directly into Vitamin C and/or 2-KGA may be selected from the D-glucose or D-sorbitol metabolization pathway such as, for example, D-glucose, D-sorbitol, L-sorbose, L-sorbosone, D-gluconate, 2-keto-D-gluconate, 2,5-diketo-gluconate or mixtures thereof. Preferably, the substrate is selected from for instance D-glucose, D-sorbitol, L-sorbose, L-sorbosone or mixtures thereof, more preferably from D-glucose, D-sorbitol, L-sorbose or mixtures thereof, and most preferably from D-sorbitol, L-sorbose or mixtures thereof.

Vitamin C as used herein may be any chemical form of L-ascorbic acid found in aqueous solutions, such as for instance undissociated, in its free acid form or dissociated as an anion. The solubilized salt form of L-ascorbic acid may be characterized as the anion in the presence of any kind of cations usually found in fermentation supernatants, such as for instance potassium, sodium, ammonium, or calcium. Also included may be isolated crystals of the free acid form of L-ascorbic acid. On the other hand, isolated crystals of a salt form of L-ascorbic acid are called by their corresponding salt name, *i. e*. sodium ascorbate, potassium ascorbate, calcium ascorbate and the like.

Conversion of a suitable carbon source into Vitamin C and/or 2-KGA in connection with the above process using a microorganism means that the conversion of the carbon source resulting in Vitamin C and/or 2-KGA is performed by the microorganism, *i.e.* the carbon source may be directly converted into Vitamin C and/or 2-KGA. Said microorganism is cultured under conditions which allow such conversion from said carbon source as it is known for the skilled person.

A medium as used herein for the above process using a microorganism may be any suitable medium for the production of Vitamin C and/or 2-KGA. Typically, the medium is an aqueous medium comprising for instance salts, substrate(s), and a certain pH.

The term "direct conversion" and the like is intended to mean that a microorganism is capable of the conversion of a certain carbon source into the specified product by means of one or more biological conversion steps, without the need of any additional chemical conversion step. For instance, the term "direct conversion of D-sorbitol into Vitamin C" is intended to describe a process wherein a microorganism is producing Vitamin C and wherein D-sorbitol is offered as a carbon source without the need of an intermediate chemical conversion step. A single microorganism capable of directly fermenting Vitamin C is preferred.

This invention is further illustrated by the following examples which should not be construed as limiting. The contents of all references, patent applications, patents and published patent applications, cited throughout this application are hereby incorporated by reference.

Examples

**Example 1: Preparation of chromosomal DNA and amplification of DNA fragment by PCR**

Chromosomal DNA of *Gluconobacter oxydans* DSM 2343 was prepared from the cells cultivated at 30°C for 1 day in VM liquid medium consisting of 5 g/l yeast extract, 3 g/l tryptone, 10 g/l mannitol, pH 6.0 using the DNeasy Tissue Kit (Qiagen, Hilden, Germany).

A DNA fragment is prepared by PCR with the chromosomal DNA prepared above and a set of primers, Pf (SEQ ID NO:3) and Pr (SEQ ID NO:4). For the reaction, the Herculase PCR kit (Stratagene, Heidelberg, Germany) and 20 ng of the chromosomal DNA is used in total volume of 50 μl according to the supplier's instruction to have the PCR product containing the pgi DNA sequence (SEQ ID NO:1). The PCR product is recovered from the reaction and its correct sequence confirmed.

Furthermore, a DNA fragment carrying both genes of pgi and tal fused together was prepared according to the method described above using the PCR primers P1 (SEQ ID NO:11) and P2 (SEQ ID NO:12) and chromosomal DNA of *Gluconobacter oxydans* DSM 2343 as template. PCR was performed as described above using the Herculase PCR kit (Stratagene, Heidelberg, Germany) and 20 ng of the chromosomal DNA resulting in a PCR product containing the pgi/tal gene (SEQ ID NO:9).

**Example 2: Overexpression of the pgi gene in *G. oxydans* DSM 17078 and *G. oxydans* IFO 3293**

The PCR product obtained in Example 1 containing the pgi/tal gene according to SEQ ID NO:9 was phosphorylated and cloned downstream of the strong G. *oxydans tufB* promoter into the dephosphorylated vector pEXGOX (SEQ ID NO:13) using *Swa*I restriction site, resulting in the vector pEXGOX-pgi/tal-fw (SEQ ID NO:14). Identity and correct orientation of the pgi/tal insert was confirmed by DNA sequence analysis.

Using *Kpn*I and *SpeI*I as restriction sites, the vector fragment including *tufB* promoter and pgi gene was cut out of pEXGOX-pgi/tal-fw and introduced into pBBR1MCS2 (Kovach et al., Gene 166:175-176, 1995) resulting in pBBR1MCS2-pgi/tal-fw (SEQ ID NO:15). The plasmid was used for transformation of *G. oxydans* DSM 17078 and *G. oxydans* IFO 3293 resulting in *G. oxydans* DSM 17078 - pBBRIMCS2-pgi/tal-fw and *G. oxydans* IFO 3293 - pBBR1MCS2-pgi/tal-fw, respectively.

Overexpression of pgi was confirmed by determination of the phosphoglucose isomerase activity in G. *oxydans* DSM 17078 - pBBRIMCS2-pgi/tal-fw and G. *oxydans* IFO 3293 - pBBR1MCS2-pgi/tal-fw compared to G. *oxydans* DSM 17078 and G. *oxydans* IFO 3293 with or without plasmid pBBR1MCS2, respectively. Individual colonies of G. *oxydans* were cultivated in 50 ml medium containing 80 g/l D-sorbitol, 20 g/l L-sorbose, 1.5 g/l $CaCl_2$, 15 g/l yeast, 2.5 g/l $MgSO_4$, 0.5 g/l glycerol, 50 μg/l cefoxitine and 50 μg/l kanamycine. The cells were grown at 30°C and harvested between OD 2.5 and 3.5, *i.e.* during mid-exponential growth phase. Activity of pgi was measured in a 1 ml sample containing 100 μl 0.5 M potassium phosphate buffer at pH 7 (Roche Diagnostics, Mannheim, Germany), 100 μl 10 mM NADP, 25 μl (200 U/ml) glucose-6-phosphate-dehydrogenase and 50 μl of cell-free extract from culture medium at OD 2.5-3.5. The reaction was started by addition of 50 μl (0.1 M) fructose-6-phosphate. An increase in NADPH formation was determined at 340 nm indicating overexpression of pgi.

In comparison to G. *oxydans* DSM 17078 with 2.04 U/ml pgi and G. *oxydans* DSM 17078-pBBRIMCS2 with 1.91 U/ml pgi, measurement of pgi activity from G. *oxydans* DSM 17078 - pBBR1MCS2-pgi/tal-fw resulted in 6.25 U/mg pgi, *i.e.* an increase of ca. 3 fold. When using G. *oxydans* IFO 3293 as host system, G. *oxydans* IFO 3293 without any plasmid contained 2.10 U/ml pgi compared to 2.07 U/ml when using G. *oxydans* IFO 3293 - pBBR1MCS2 and 5.85 U/ml pgi with G. *oxydans* IFO 3293 - pBBRIMCS2-pgi/tal-fw, thus an increase of ca. 3 fold in pgi activity.

Strains containing the pgi gene alone, *i.e. G. oxydans* DSM 17078 - pBBRIMCS2-pgi-fw and G. *oxydans* IFO 3293 - pBBR1MCS2-pgi-fw, respectively, are prepared accordingly and tested for pgi overexpression against G. *oxydans* DSM 17078, G. *oxydans* DSM 17078 - pBBR1MCS2, G. *oxydans* IFO 3293 and G. *oxydans* IFO 3293 - pBBR1MCS2, respectively. As a result, an increase of at least 3 fold is detected.

**Example 3: Biomass production upon overexpression of pgi in *G. oxydans* DSM 17078 and *G. oxydans* IFO 3293 in liquid culture**

Biomass formation from D-sorbitol was tested in shake flasks experiments. Growth of G. *oxydans* DSM 17078 - pBBRIMCS2 and *G. oxydans* IFO 3293 - pBBRIMCS2, respectively, was compared to a strain overexpressing pgi according to Examples 1 and 2. Biomass formation was determined via measuring the OD. The following medium was used: 15 g/l yeast extract, 0.5 g/l glycerol, 2.5 g/l $MgSO_4$, 1.5g/l $CaCl_2$, 80g/l D-sorbitol, 20g/l L-sorbose, 50 $\mu$g/l cefoxitine, 50 $\mu$g/l kanamycine. 50 ml medium in 500ml non-beveled Erlenmeyer flasks were inoculated to an OD of 0.2 from a 24h pre-culture in the same medium. The culture was incubated at 30°C at 200 rpm shaking.

The OD after 68 h improved from 6.01 for DSM 17078 - pBBRIMCS2 to 9.51 for DSM 17078 - pBBR1MCS2-pgi/tal-fw, corresponding to an increase of 58% in biomass formation. *G. oxydans* IFO 3293 as host, the OD after 68 h improved from 5.12 for G. *oxydans* IFO 3293 - pBBRIMCS2 to 7.18 for G. *oxydans* IFO 3293 - pBBR1MCS2-pgi/tal-fw, corresponding to an increase of 40% in biomass formation.

Improvement of yield and productivity of biomass formation in liquid culture by overexpression of pgi in G. *oxydans* DSM 17078 was confirmed by comparing growth on D-sorbitol for G. *oxydans* DSM 17078 - pBBRIMCS2-pgi/tal-fw and G. *oxydans* DSM 17078 - pBBR1MCS2 in a fermenter. Following medium was used: 15 g/l yeast; 2,5 g/l $MgSO_4$; 0.5 g/l glycerol; 100 g/l D-sorbitol; 1.5 g/l $CaCl_2$; 50 $\mu$g/l cefoxitine; 50 $\mu$g/l kanamycine; one drop antifoam. Fermentations conditions were: 200 ml medium per fermenter, N = 500 rpm, $pO_2$ = 20%, aeration rate: 15 sl/h, $X_{CO2}$ n the air: 0,0 %, pH = 6.0, inocculation from seed to OD=0.5. Two independent fermentation runs were performed from two different seeds for both G. *oxydans* DSM 17078 - pBBR1MCS2 and G. *oxydans* DSM 17078 - pBBRIMCS2-pgi-fw. Growth of biomass was followed over 102 h by determination of the optical density at 600 nm ($OD_{600}$).

Overexpression of pgi in *G. oxydans* DSM 17078 results in an improved productivity as well in an increase of the yield of biomass formation of *G. oxydans* DSM 17078 from D-sorbitol by ca. 47%: The $OD_{600}$ after 102 hours was 33.0 for *G. oxydans* DSM 17078 - pBBR1MCS2-pgi/tal-fw compared to 22.5 for *G. oxydans* DSM 17078 - pBBRIMCS2. In all fermentations for *G. oxydans* DSM 17078 - pBBRIMCS2-pgi/tal-fw and DSM 17078 - pBBR1MCS2, the carbon source D-sorbitol was completely consumed after 102 hours.

An increase of about 40, 50, 60% in yield of biomass production from D-sorbitol is also obtained in experiments according to the method described above using G. *oxydans* DSM 17078 - pBBR1MCS2-pgi-fw compared to strain G. *oxydans* DSM 17078 - pBBRIMCS2.

**Example 4: Vitamin C production upon overexpression of pgi in *G. oxydans* DSM 17078 using resting cells**

Cells of *G. oxydans* DSM 17078, *G. oxydans DSM 17078* - pBBR1MCS2-pgi-fw are grown at 27°C for 3 days on No. 3BD agar medium containing 70 g/l D-sorbitol, 0.5 g/l glycerol, 7.5 g/l yeast extract (Difco), 2.5 g/l $MgSO_4 \cdot 7H_2O$, 10 g/l $CaCO_3$ and 18 g/l agar (Difco).

Cells are scraped from the agar plates, suspended in distilled water and used for resting cell reactions conducted at 30°C with shaking at 220 rpm. A series of reactions (0.5 ml reaction mixture in 5 ml reaction tubes) are carried out with 2% D-sorbitol in reaction mixtures further containing 0.3% NaCl, and 1% $CaCO_3$ and is incubated with cells at a final concentration of $OD_{600}$ = 10. After an incubation period of 20 hours, samples of the reaction mixtures are analyzed by high performance liquid chromatography (HPLC) using an Agilent 1100 HPLC system (Agilent Technologies, Wilmington, USA) with a LiChrospher-100-RP18 (125 x 4.6 mm) column (Merck, Darmstadt, Germany) attached to an Aminex-HPX-78H (300 x 7.8 mm) column (Biorad, Reinach, Switzerland). The mobile phase is 0.004 M sulfuric acid, and the flow rate was 0.6 ml/min. Two signals are recorded using an UV detector (wavelength 254 nm) in combination with a refractive index detector. In addition, the identification of the L-ascorbic acid is done using an amino-column (YMC-Pack Polyamine-II, YMC, Inc., Kyoto, Japan) with UV detection at 254 nm. The mobile phase is 50 mM $NH_4H_2PO_4$ and acetonitrile (40:60).

An Agilent Series 1100 HPLC-mass spectrometry (MS) system is used to identify L-ascorbic acid. The MS is operated in positive ion mode using the electrospray interface. The separation is carried out using a LUNA-C8(2) column (100 x 4.6 mm) (Phenomenex, Torrance, USA). The mobile phase is a mixture of 0.1 % formic acid and methanol (96: 4). L-Ascorbic acid elutes with a retention time of 3.1 minutes. The identity of the L-ascorbic acid is confirmed by retention time and the molecular mass of the compound.

The supernatants of the reaction mixtures incubated with cells of G. *oxydans* DSM 17078 - pBBR1MCS2-pgi-fw contain at least 20% more Vitamin C than the supernatant of the reaction mixture incubated with cells of G. *oxydans* DSM 17078.

## Claims

1. A process for the production of biomass from a carbon source wherein a microorganism which is cultivated in an aqueous nutrient medium under conditions that allow the growth on said carbon source, said microorganism being genetically engineered by a polynucleotide selected from the group consisting of:

   (a) polynucleotides encoding a polypeptide comprising the amino acid sequence according to SEQ ID NO:2;
   (b) polynucleotides comprising the nucleotide sequence according to SEQ ID NO:1;
   (c) polynucleotides comprising a nucleotide sequence obtainable by nucleic acid amplification such as polymerase chain reaction, using genomic DNA from a microorganism as a template and a primer set according to SEQ ID NO:3 and SEQ ID NO:4;
   (d) polynucleotides comprising a nucleotide sequence encoding a fragment or derivative of a polypeptide encoded by a polynucleotide of any of (a) to (c) wherein in said derivative one or more amino acid residues are conservatively substituted compared to said polypeptide, and said fragment or derivative has the activity of an isomerase [EC 5], preferably an intramolecular oxidoreductase [EC 5.3], most preferably pgi;
   (e) polynucleotides the complementary strand of which hybridizes under stringent conditions to a polynucleotide as defined in any one of (a) to (d) and which encode an isomerase [EC 5], preferably an intramolecular oxidoreductase [EC 5.3], most preferably pgi;
   (f) polynucleotides which are at least 70%, such as 85, 90 or 95% identical to a polynucleotide as defined in any one of (a) to (d) and which encode an isomerase [EC 5], preferably an intramolecular oxidoreductase [EC 5.3], most preferably pgi;

   or
   the complementary strand of such a polynucleotide.

2. Process for the production of a microorganism capable of growth on a given carbon source, comprising the step of altering said microorganism so that the microorganism produces a polypeptide with increased and/or improved isomerase [EC 5], preferably intramolecular oxidoreductase [EC 5.3], most preferably pgi activity leading to an improved yield and/or production of biomass from said carbon source by said microorganism.

3. Process for the production of a microorganism containing an endogenous gene comprising a polynucleotide as described in claim 1, comprising the step of altering said microorganism so that the endogenous gene is overexpressed, leading to an improved yield and/or production of biomass from a carbon source produced by said microorganism.

**4.** A microorganism genetically engineered with a polynucleotide selected from the group consisting of:

(a) polynucleotides encoding a polypeptide comprising the amino acid sequence according to SEQ ID NO:2;
(b) polynucleotides comprising the nucleotide sequence according to SEQ ID NO:1;
(c) polynucleotides comprising a nucleotide sequence obtainable by nucleic acid amplification such as polymerase chain reaction, using genomic DNA from a microorganism as a template and a primer set according to SEQ ID NO:3 and SEQ ID NO:4;
(d) polynucleotides comprising a nucleotide sequence encoding a fragment or derivative of a polypeptide encoded by a polynucleotide of any of (a) to (c) wherein in said derivative one or more amino acid residues are conservatively substituted compared to said polypeptide, and said fragment or derivative has the activity of an isomerase [EC 5], preferably an intramolecular oxidoreductase [EC 5.3], most preferably pgi;
(e) polynucleotides the complementary strand of which hybridizes under stringent conditions to a polynucleotide as defined in any one of (a) to (d) and which encode an isomerase [EC 5], preferably an intramolecular oxidoreductase [EC 5.3], most preferably pgi;
(f) polynucleotides which are at least 70%, such as 85, 90 or 95% identical to a polynucleotide as defined in any one of (a) to (d) and which encode an isomerase [EC 5], preferably an intramolecular oxidoreductase [EC 5.3], most preferably pgi;

or
the complementary strand of such a polynucleotide.

**5.** The microorganism according to claim 4 wherein said polynucleotide sequence is present in form of a vector.

**6.** The microorganism according to claim 4, said microorganism being genetically altered in such a way that it leads to an improved yield and/or efficiency of biomass from a carbon source produced by said microorganism.

**7.** The microorganism according to any one of claims 4 to 6 capable of producing biomass from sorbitol in a yield of at least about 0.5 g biomass/g sorbitol.

**8.** The microorganism according to claim 7 wherein the yield is at least about 0.2 g biomass/g sorbitol.

**9.** A polynucleotide selected from the group consisting of:

(a) polynucleotides encoding a polypeptide comprising the amino acid sequence according to SEQ ID NO:2;
(b) polynucleotides comprising the nucleotide sequence according to SEQ ID NO: 1;
(c) polynucleotides comprising a nucleotide sequence obtainable by nucleic acid amplification such as polymerase chain reaction, using genomic DNA from a microorganism as a template and a primer set according to SEQ ID NO:3 and SEQ ID NO:4;
(d) polynucleotides comprising a nucleotide sequence encoding a fragment or derivative of a polypeptide encoded by a polynucleotide of any of (a) to (c) wherein in said derivative one or more amino acid residues are conservatively substituted compared to said polypeptide, and said fragment or derivative has the activity of an isomerase [EC 5], preferably an intramolecular oxidoreductase [EC 5.3], most preferably pgi;
(e) polynucleotides the complementary strand of which hybridizes under stringent conditions to a polynucleotide as defined in any one of (a) to (d) and which encode an isomerase [EC 5], preferably an intramolecular oxidoreductase [EC 5.3], most preferably pgi;
(f) polynucleotides which are at least 70%, such as 85, 90 or 95% identical to a polynucleotide as defined in any one of (a) to (d) and which encode an isomerase [EC 5], preferably an intramolecular oxidoreductase [EC 5.3], most preferably pgi;

or
the complementary strand of such a polynucleotide.

**10.** A vector containing the polynucleotide according to claim 9.

**11.** A polypeptide encoded by a polynucleotide according to claim 9.

**12.** A process for producing cells capable of expressing a polypeptide according to claim 11, comprising the step of genetically engineering cells with the vector of claim 10 or with a polynucleotide according to claim 9.

**13.** Use of a polynucleotide according to claim 9 or a vector according to claim 10 for the production of biomass from a carbon source.

**14.** Use of a polynucleotide according to claim 9 or a vector according to claim 10 for the production of Vitamin C from a carbon source.

**15.** Use according to claim 13 or 14, wherein the polynucleotide is operatively linked to expression control sequences and transferred into a microorganism.

**16.** Use according to claim 15, wherein the expression control sequences comprise a regulation-, and/or promoter-, and/or terminator sequence and wherein at least one of these sequences is altered in such a way that it leads to an improved yield and/or production of biomass from a carbon source produced by said microorganism.

**17.** Use according to claim 15, wherein the expression control sequences comprise a regulation-, and/or promoter-, and/or terminator sequence and wherein at least one of these sequences is altered in such a way that it leads to an improved yield and/or production of Vitamin C from a carbon source produced by said microorganism.

**18.** Use according to claim 16 or 17, wherein the expression control sequences comprise a regulation-, and/or promoter-, and/or terminator sequence and wherein at least one of these sequences is altered in such a way that it leads to an increased and/or improved activity of an isomerase [EC 5], preferably an intramolecular oxidoreductase [EC 5.3], most preferably pgi.

**19.** The microorganism according to any one of claims 4 to 8 producing a polypeptide according to claim 11 with increased and/or improved isomerase [EC 5], preferably intramolecular oxidoreductase [EC 5.3], most preferably pgi activity.

**20.** The microorganism according to claim 19 wherein the polynucleotide according to claim 9 is overexpressed.

**21.** The microorganism according to any one of claims 4 to 8, 19 or 20 selected from the group consisting of *Pseudomonas, Pantoea, Escherichia, Corynebacterium, Ketogulonicigenium* and acetic acid bacteria like *e.g., Gluconobacter, Acetobacter* or *Gluconacetobacter,* preferably *Acetobacter* sp., *Acetobacter aceti, Gluconobacter frateurii, Gluconobacter cerinus, Gluconobacter thailandicus, Gluconobacter oxydans,* preferably *Gluconobacter oxydans,* more preferably *Gluconobacter oxydans* DSM 17078.

**22.** Process for the production of an enhanced endogenous isomerase [EC 5], preferably intramolecular oxidoreductase [EC 5.3], most preferably pgi gene in a microorganism, said microorganism comprising a polynucleotide according to claim 9, said process comprising the step of altering said polynucleotide in such a way that it leads to an improved yield and/or efficiency of biomass production produced from a carbon source by said microorganism.

**23.** Process according to any one of claims 1 to 3, 12 or 22 or use according to any one of claims 13 to 18 wherein the carbon source is selected from the group consisting of sorbitol, glucose, fructose, mannitol, sorbose, glycerol, sucrose, maltose, starch and mixtures thereof.

**24.** The microorganism according to any one of claims 6 to 8, 19 or 21 wherein the carbon source is selected from the group consisting of glucose, fructose, sorbitol, mannitol, sorbose, glycerol, sucrose, maltose, starch, and mixtures thereof.

European Patent Office

# EUROPEAN SEARCH REPORT

Application Number
EP 06 01 3092

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE UniProt [Online] 1 March 2005 (2005-03-01), PRUST C ET AL: "Transaldolase (EC 2.2.1.2) Gluconobacter oxydans (Gluconobacter suboxydans)" XP002410002 retrieved from EBI Database accession no. Q5FQA2 * the whole document * | 11 | INV. C12N15/61 C12N9/92 C12P17/04 C12P1/04 C12N1/20 |
| A | -& PRUST C ET AL: "Complete genome sequence of the acetic acid bacterium Gluconobacter oxydans" NATURE BIOTECHNOLOGY, vol. 23, no. 2, February 2005 (2005-02), pages 195-200, XP002334906 ISSN: 1087-0156 * abstract * * page 195, left-hand column, line 1 - right-hand column, line 6 * * figure 1 * ----- | 7-9,11, 14,17, 23,24 | |
| X | DATABASE Geneseq [Online] 20 May 2004 (2004-05-20), "Gluconobacter oxydans glucose-6-phosphoric acid isomerase protein." XP002410003 retrieved from EBI accession no. GSP:ADL90006 Database accession no. ADL90006 * the whole document * | 11 | TECHNICAL FIELDS SEARCHED (IPC): C12N C12P |
| X | -& DATABASE WPI Week 200413 Derwent Publications Ltd., London, GB; AN 2004-127093 XP002410012 & JP 2004 024140 A (AJINOMOTO KK) 29 January 2004 (2004-01-29) * abstract * ----- -/-- | 1-6, 9-13,15, 16,18-24 | |

The present search report has been drawn up for all claims

| Place of search | Date of completion of the search | Examiner |
|---|---|---|
| The Hague | 1 December 2006 | van de Kamp, Mart |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 06 01 3092

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE UniProt [Online] 5 July 2004 (2004-07-05), SUGIYAMA M ET AL: "Transaldolase and glucose-6-phosphate isomerase bifunctional protein Gluconobacter oxydans (Gluconobacter suboxydans)" XP002410004 retrieved from EBI Database accession no. Q76EM6 * the whole document * | 11 | |
| X | DATABASE EMBL [Online] 24 January 2005 (2005-01-24), XP002410005 retrieved from EBI accession no. EM_PRO:CP000009_3407 Database accession no. CP000009 * the whole document * | 9,11 | |
| X | DATABASE Geneseq [Online] 20 May 2004 (2004-05-20), "Gluconobacter oxydans NADH production related coding sequence." XP002410006 retrieved from EBI accession no. GSN:ADL90004 Database accession no. ADL90004 * the whole document * | 9 | TECHNICAL FIELDS SEARCHED (IPC) |

-/--

The present search report has been drawn up for all claims

| Place of search | Date of completion of the search | Examiner |
|---|---|---|
| The Hague | 1 December 2006 | van de Kamp, Mart |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

7

EPO FORM 1503 03.82 (P04C01)

European Patent Office

# EUROPEAN SEARCH REPORT

Application Number
EP 06 01 3092

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE EMBL [Online] 3 February 2004 (2004-02-03), SUGIYAMA M ET AL: "Gluconobacter oxydans tkt, tal-pgi, gnd genes for transketolase, transaldolase and glucose-6-phosphate isomerase bifunctional protein, 6-phosphogluconate dehydrogenase, complete cds." XP002410007 retrieved from EBI accession no. EM_PRO:AB106333 Database accession no. AB106333 * the whole document * ----- | 9,11 | |
| X | JP 2004 024140 A (AJINOMOTO KK) 29 January 2004 (2004-01-29) * the whole document * ----- | 1-6, 9-13,15, 16,18-24 | |
| X | SUGIYAMA M ET AL: "Transaldolase/glucose-6-phosphate isomerase bifunctional enzyme and ribulokinase as factors to increase xylitol production from D-arabitol in Gluconobacter oxydans." BIOSCIENCE BIOTECHNOLOGY AND BIOCHEMISTRY, vol. 67, no. 12, December 2003 (2003-12), pages 2524-2532, XP002410001 ISSN: 0916-8451 * the whole document * * page 2526, right-hand column, line 31 - page 2527, left-hand column, line 8 * * page 2528, right-hand column, line 4 - page 2529, right-hand column, line 11 * * page 2531, right-hand column, lines 29-32 * ----- -/-- | 1-6, 9-13,15, 16,18-24 | TECHNICAL FIELDS SEARCHED (IPC) |

The present search report has been drawn up for all claims

| Place of search | Date of completion of the search | Examiner |
|---|---|---|
| The Hague | 1 December 2006 | van de Kamp, Mart |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

7

EPO FORM 1503 03.82 (P04C01)

European Patent Office

# EUROPEAN SEARCH REPORT

Application Number
EP 06 01 3092

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KARHUMAA KAISA ET AL: "Investigation of limiting metabolic steps in the utilization of xylose by recombinant Saccharomyces cerevisiae using metabolic engineering" YEAST, vol. 22, no. 5, April 2005 (2005-04), pages 359-368, XP002347259 ISSN: 0749-503X * abstract * * figure 2 * | 2 | |
| X | WO 2006/009434 A (UNIV DELFT TECH [NL]; WINKLER AARON ADRIAAN [NL]; KUYPER SIPKO MAARTEN) 26 January 2006 (2006-01-26) * example 1 * * claims 2,3 * | 2 | |
| X | WO 2005/113759 A2 (BIOTECHNOLOGY RES & DEV [US]; ARGRICULTURAL RES SERVICE UNIT [US]; FOT) 1 December 2005 (2005-12-01) * examples 1,2 * * claims 1-6,10,18 * | 2 | TECHNICAL FIELDS SEARCHED (IPC) |
| A | DEPPENMEIER U ET AL: "Biochemistry and biotechnological applications of Gluconobacter strains" APPLIED MICROBIOLOGY AND BIOTECHNOLOGY, vol. 60, no. 3, November 2002 (2002-11), pages 233-242, XP002334995 ISSN: 0175-7598 * the whole document * * figure 2 * * page 237, left-hand column, line 3 - page 238, right-hand column, line 32 * | | |

The present search report has been drawn up for all claims

| Place of search | Date of completion of the search | Examiner |
|---|---|---|
| The Hague | 1 December 2006 | van de Kamp, Mart |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

7

## ANNEX TO THE EUROPEAN SEARCH REPORT ON EUROPEAN PATENT APPLICATION NO. EP 06 01 3092

This annex lists the patent family members relating to the patent documents cited in the above-mentioned European search report.
The members are as contained in the European Patent Office EDP file on
The European Patent Office is in no way liable for these particulars which are merely given for the purpose of information.

01-12-2006

| Patent document cited in search report | | Publication date | Patent family member(s) | Publication date |
|---|---|---|---|---|
| JP 2004024140 | A | 29-01-2004 | NONE | |
| WO 2006009434 | A | 26-01-2006 | NONE | |
| WO 2005113759 | A2 | 01-12-2005 | US 2006110805 A1 | 25-05-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SAMBROOK et al.** Molecular Cloning, A Laboratory Manual. Cold Spring Harbor Press, 1989
- Current Protocols in Molecular Biology. John Wiley & Sons, 1995
- **SUGISAWA et al.** *Agric. Biol. Chem.,* 1990, vol. 54, 1201-1209
- **SAMBROOK et al.** Molecular Cloning : A Laboratory Manual. Cold Spring Harbor Laboratory, Cold Spring Harbor Laboratory Press, 1989
- **NEEDLEMAN ; WUNSCH.** *J. Mol. Biol.,* 1970, 444-453
- **E. MEYERS ; W. MILLER.** *CABIOS,* 1989, vol. 4, 11-17
- **ALTSCHUL et al.** *J. Mol. Biol.,* 1990, vol. 215, 403-10
- **ALTSCHUL et al.** *Nucleic Acids Res.,* 1997, vol. 25 (17), 3389-3402
- **GOEDDEL.** Gene Expression Technology: Methods in Enzymology. Academic Press, 1990
- **DAVIS et al.** *Basic Methods in Molecular Biology,* 1986
- **SUGIYAMA et al.** *Biosci. Biotechnol. Biochem.,* 2003, vol. 67 (12), 2524-2532
- **KOVACH et al.** *Gene,* 1995, vol. 166, 175-176